# EUROPÄISCHE PATENTSCHRIFT

(11) **EP 2 494 951 B1**
(45) Veröffentlichungstag und Bekanntmachung des Hinweises auf die Patenterteilung: **28.06.2017**
(21) Anmeldenummer: 12001265.3
(22) Anmeldetag: 27.02.2012
(51) Int. Cl.: A61K 6/10

(54) **Lager- und formstabile dentale elastomere Abformmaterialien hoher Viskosität**
Highly viscous dental elastomeric impression materials which are storage stable and form stable
Matériau de prise d'enpreinte dentaire en élastomère hautement visqueux stable au stockage et en forme

(30) Priorität: 01.03.2011 DE 102011012745
(43) Veröffentlichungstag der Anmeldung: 05.09.2012
(73) Patentinhaber: VOCO GmbH, 27472 Cuxhaven (DE)
(72) Erfinder: Riedel, Norman, Hendrik, 60435 Frankfurt am Main (DE); Krumme, Wigand, 27476 Cuxhaven (DE); Dr. Blömker, Tobias, 22527 Hamburg (DE); Plaumann, Manfred, Thomas, 27472 Cuxhaven (DE)
(74) Vertreter: Hauck Patentanwaltspartnerschaft mbB

(56) Entgegenhaltungen:
- WO-A2-2008/059468
- US-A- 4 879 339
- US-A- 5 066 714
- US-A1- 2004 110 863

## Beschreibung

Die vorliegende Erfindung betrifft dentale elastomere Abformmaterialien hoher, bzw. sehr hoher Konsistenz, deren Abformscheiben gemäß ISO 4823 einen Durchmesser von maximal 35 mm aufweisen.

Die Konsistenzprüfung nach der ISO-Norm erfolgt dabei anhand einer Messung mit zwei Glasplatten, wobei auf der Basisscheibe 0,5 ml des zu testenden Elastomers appliziert und anschließend mit Hilfe einer zweiten Platte unter Einwirkung einer vorgegebenen Kraft ausgepresst wird. Die Konsistenz wird mit Hilfe des durchschnittlichen Durchmessers der resultierenden Abformscheibe in unterschiedliche Typen unterteilt.

Die vorliegende Erfindung betrifft bevorzugt Materialien der Typklasse 0, also hochviskose oder knetbare Materialien, die auch Puttymassen oder Puttys genannt werden.

Während der Abformung von Zähnen muss das Abformmaterial in einem plastischen Zustand in die Mundhöhle gebracht werden, wo es eine Phasenumwandlung erfährt und in einen festelastischen oder starren Zustand übergeht, so dass es möglich ist, den Abdruck in fester Form und ohne Änderungen seiner Form aus der Mundhöhle wieder zu entfernen.

Die vorliegende Erfindung betrifft demnach dentale Puttys auf der Basis additionsvernetzender Silikone. Diese Substanzklasse, bei der es sich um kaltvernetzende Zweikomponentensysteme handelt, bei denen zwei Pasten miteinander vermengt werden, die bei Raumtemperatur nach wenigen Minuten miteinander aushärten, zeichnet sich durch eine äußerst geringe Schrumpfung während der Vernetzung aus und gibt die abzuformende Situation am Patienten in der Regel dimensions- und detailgetreu wieder.

Dentale Puttymassen auf der Basis additionsvernetzender Silikone werden zum einen häufig in einer sogenannten Korrekturabformung eingesetzt. Hierbei wird mit dem schwerfließenden oder knetbaren elastischen Silikonmaterial eine erste Abformung (Vorabformung) im Löffel durchgeführt. Bei Anfertigung der Vorabformung ist es wünschenswert, dass das Abformmaterial standfest ist und so dem Einsetzen des mit dem Putty gefüllten Abformlöffels in den Mund des Patienten einen Widerstand entgegensetzt. Das Einsetzen des Löffels wird durch den Widerstand gebremst, wodurch ein Durchdrücken des Löffels bis zu den Zahnoberflächen vermieden werden kann. Ein Kontakt des Löffelbodens mit den Zahnoberflächen ist für den Patienten schmerzhaft und führt zu fehlerhaften Formen, da an den Kontaktflächen kein Abformmaterial mehr vorhanden ist.

Nach Entnahme der Abformung aus dem Mund wird der Abdruck zur Beseitigung von Unterschnitten und zur Schaffung von Abflussmöglichkeiten für das dünnflie-ßende Silikonmaterial bei der zweiten Abformung (Korrekturabformung) beschnitten. Mit der zweiten Abformung wird die Vorabformung korrigiert, um die Detailschärfe des Erstabdrucks im cervikalen und subgingivalen Bereich zu erhöhen. Hierbei wird der Zahnkranz des ersten Abdrucks im Löffel mit einem niedrigviskosen Material gleicher chemischer Natur ausgefüllt. Gegebenenfalls wird der präparierte Zahn zusätzlich umspritzt. Durch Wiedereinsetzen des Löffels wird das Korrekturmaterial bis auf eine dünne Korrekturschicht verdrängt. Es verbindet sich mit dem polymerisierten Erstmaterial und härtet ebenfalls aus. Bei dem Eindringen des Zahns/Zahnstumpfs in den mit dünnfließendem Material gefüllten Hohlraum der Erstabformung wird ein ausreichender Druck erzeugt, der sicherstellt, dass das Material auch in schwer zugängliche Bereiche gepresst wird, so dass auch kleinste Details des Präparats exakt wiedergegeben werden.

Die oben beschriebene Korrekturabformung wird auch als zweizeitige Zweiphasenabformung bezeichnet, da das Verfahren mit zwei Materialien unterschiedlicher Viskosität bei gleicher chemischer Basis sukzessiv erfolgt.

Dentale Puttymassen auf der Basis additionsvernetzender Silikone werden zum anderen auch häufig in einer sogenannten Doppelmischabformung eingesetzt. Hierbei wird der präparierte Zahn im Mund des Patienten mit dünnfließendem Abformmaterial umspritzt. Solange das Material noch fließfähig ist, wird jetzt der Löffel mit dem Putty im Mund platziert. Beide Materialphasen binden dann gemeinsam zu einem Abdruck ab.

Die Doppelmischabformung wird auch einzeitige Zweiphasenabformung bezeichnet, da hier zur gleichen Zeit der Löffel mit dem Putty befüllt und der Zahn/Zahnstumpf mit dem niedrigviskosen Silikon umspritzt wird.

Die vorliegende Erfindung betrifft ferner Zusammensetzungen zur Herstellung dentaler Puttymassen sowie Verfahren zur Herstellung dentaler Abdrücke unter Verwendung erfindungsgemäßer Puttys, sie betrifft zudem die dentalen Abdrücke selbst, die durch Vernetzung der erfindungsgemäßen Puttys herstellbar sind. Gegenstand der Erfindung ist ebenfalls ein Kit zur Herstellung dentaler Abdrücke, umfassend erfindungsgemäße Puttys. Schließlich betrifft die Erfindung die Verwendung eines erfindungsgemäßen Puttys zum Herstellen dentaler Abdrücke.

Bei ihrer Anwendung werden die beiden Pasten mit Dosierlöffeln aus ihren Lagerbehältern entnommen und die möglichst gleich großen Portionen mit den Händen zu einer homogenen Masse verknetet. Diese Masse wird in einen Abformlöffel gegeben und gemäß der oben beschriebenen Methoden verwendet. Die Masse härtet innerhalb weniger Minuten aus und kann dann aus dem Mund des Patienten entfernt werden. Aus dem Polymerisat wird dann der Abdruck hergestellt.

Bei der Entnahme der Pasten aus den Lagerbehältern sowie beim Mischen der Paste durch Kneten mit den Fingern ist es wichtig, dass die Masse nicht an den Entnahmelöffeln bzw. den Fingern klebt oder Rückstände hinterlässt. Zu einem gewissen Grad kann dies durch den Zusatz geeigneter Füllstoffe und/oder inerter Zusätze in Form von Kohlenwasserstoffen, beispielsweise von Paraffinölen in einer genau abgestimmten Menge erreicht werden. Problematisch an diesen Puttyzusammensetzungen ist jedoch die Beobachtung, dass derart zusammengesetzte Pasten sowohl während der Lagerung als auch nach der Vernetzung große Anteile der Kohlenwasserstoffe, die frei und chemisch nicht gebunden in den Zusammensetzungen vorliegen, aus der Masse herauswandern und freigesetzt werden. Dieses Phänomen bezeichnet man auch als Ausschwitzen, da sich hierbei Tröpfchen des Zusatzes an den Oberflächen der beiden Pasten oder später auch an den Oberflächen der Abformung bilden.

Das Ausschwitzen der Zusätze, die die Puttymasse erst knetbar und gut verarbeitbar machen, kann ganz entscheidende Auswirkungen auf die Qualität der Abformung haben:
Die an die Oberfläche abgewanderten und aus dem Verbund des Silikonnetzwerks abgeschiedenen Kohlenwasserstoffe können die Adhäsion zwischen der vernetzten Puttymasse und dem niedrigviskosen Korrekturmaterial massiv stören, bzw. ganz unterbinden. Darüberhinaus kann durch das Ausschwitzen die Haftung der Putty-masse an den Wandungen des Abformlöffels verringert werden, so dass eine klare und detailgenaue Abformung nicht zustande kommen kann.

Es gibt im Stand der Technik einige Bemühungen, die o.g. Probleme zu lösen und leicht knetbare Puttymassen, die ihre Zusätze nicht freigeben und ausschwitzen, zu offenbaren.

Das Patent EP 0 152 887 B1 trägt den Titel "Modifizierte Füllstoffe für Silikonpasten, deren Verwendung und diese Füllstoffe enthaltende Dentalabformmassen" und offenbart den Einsatz von paraffinbeladenen Füllstoffen in den Pasten. Um die Klebrigkeit in den Pasten zu eliminieren, sollen vorzugsweise nur noch etwa 4 - 5 Gew.-% Paraffinöl in der Paste, wovon sich jedoch etwa 1 - 2 Gew.-% Paraffinöl bereits auf der Oberfläche der Füllstoffe befinden, benötigt werden. Die Pasten sollen demnach nur noch etwa 2 - 4 Gew.-% freies Paraffinöl enthalten, welches wiederum Affinität zum paraffinölbeladenen Füllstoff besitzt und weniger zum Ausschwitzen neigen soll. Auf diese Weise sollen lagerstabilere Puttys bzw. Abformungen erhalten werden. Es sollen sich kaum noch Tröpfchen von Paraffinöl in den Pasten bzw. auf den Abformungen bilden.

In der EP 0 166 107 A2 mit dem Titel "Verwendung von Paraffin- oder Mikrowachsen für Silikonpasten, deren Konfektionierung und Verwendung" wird angegeben, dass die benannten Probleme durch den Einsatz von Paraffin- oder Mikrowachsen in den Pasten vermieden werden können. Um die Klebrigkeit der Pasten zu eliminieren sollen vorzugsweise nur noch etwa 4 - 5 Gew.-% Paraffinöl und 1,5 - 5 Gew.-% Paraffin- oder Mikrowachs benötigt werden. Die Wachse sind bei RT fest, da sie einen Schmelzbereich zwischen 30 - 55°C aufweisen. Sie werden in die Massen durch Energiezufuhr beim Kneten während der Pastenherstellung eingearbeitet. Das Paraffinöl soll in diesen Pasten weniger zum Ausschwitzen neigen und man soll auf diese Weise lagerstabilere Puttys bzw. Abformungen erhalten. Es sollen sich kaum noch Tröpfchen von Paraffinöl in den Pasten bzw. auf den Abformungen bilden.

Die EP 0 158 141 B1 ("Platin, Organopolysiloxan und Füllstoff enthaltende Pasten") beschreibt Dentalvorabdruckmassen, die sich besonders rasch und leicht vermischen lassen sollen und keine oder praktisch keine Spuren hinterlassen sollen, wenn die Pasten mit den bloßen Händen unter Kneten vermischt werden. Die Katalysatorpaste enthält 10 bis 25 Gew.-% von aliphatischen C-C-Mehrfachbindungen freie, bei RT flüssige oder verstreichbare Kohlenwasserstoffe. Bevorzugt werden Paraffinöle und Petroleum eingesetzt. Das Ausschwitzen der Kohlenwasserstoffe wurde nicht untersucht.

Die EP 0 219 660 B1, benannt "Silikonabformmassen", nimmt Bezug auf die oben referierten Druckschriften und erläutert, dass aus diesen Schriftstücken bekannt geworden sei, dass Puttys, die n-Paraffine enthalten, die Klebrigkeit der Massen an den Händen des Verarbeiters reduzieren. Allerdings sollen derartige Pasten als auch die daraus hergestellten Abformungen während der Lagerung einen Teil des Paraffins ausschwitzen, wodurch Abformungen mit nicht ausreichender Präzision erhalten werden.

In der Patentschrift wird weiter ausgeführt, dass bei dem Bemühen, die Puttymassen weiter zu verbessern, gefunden wurde, dass Modifikationen der Massen mit Isoparaffinen mit 8 - 24 C-Atomen zu hochwertigen Puttys führen. Die Massen sollen sich durch ihre Lagerstabilität und Gleitfähigkeit beim Vermischen von Basis- und Katalysatorpaste auszeichnen. Sie sollen sich insbesondere für die Herstellung genauer Abformungen, speziell auch von Zähnen, eignen. Bevorzugt eingesetzte Isoparaffine sind Isohexadecan und Isoeikosan.

Die inerten Kohlenwasserstoffe, die den dentalen Puttymassen als Additive zur Reduktion der Klebrigkeit bei Handmischung der beiden Komponenten zugegeben werden, werden auch als Trennmittel (release agent) oder Weichmacher (plasticizer) bezeichnet. Die Wirksamkeit ihrer Beigabe zur dentalen Masse betrifft nicht nur ein einfaches und klebefreies Kneten der beiden Komponenten mit den Händen. Die nicht klebrige Konsistenz führt auch dazu, dass das gemischte Material im Abformlöffel noch manipuliert werden kann, beispielsweise um eine größere Pastenmenge an eine gewünschte Stelle bewegen zu können.

Darüber hinaus verleihen die Additive den Pasten eine besondere Geschmeidigkeit, die das Vermischen der beiden Komponenten untereinander vereinfacht, d.h. beschleunigt.

Die obige "Putty-Problematik" wird abermals in der US 4,879,339 ("Storage stable and room temperature curable organopolysiloxan composition") aufgenommen. Es heißt dort, daß zur Reduzierung der Klebrigkeit den dentalen knetbaren und standfesten Abformmasssen typischerweise flüssige bis halbfeste aliphatische Kohlenwasserstoffe als Trennmittel beigegeben werden. Beispielhaft werden flüssige Paraffine und Petrolatum genannt.

In Gegenwart des Platinkatalysators seien die Kohlenwasserstoffe jedoch anfällig gegenüber einer katalytischen Oxidation, wobei sich unterschiedlich oxidierte Gruppen wie Carbonsäuren, Aldehyde oder Hydroperoxide bilden könnten, die ihrerseits zumindest teilweise den Platin-Katalysator deaktivieren und abbauen könnten. Dies wiederum könnte zu einer verzögerten Aushärtung oder zu nicht vernetzten Monomeranteilen im Polymerisat führen.

In der Patentschrift wird demnach vorgeschlagen, der Puttymasse Antioxidantien in einer Menge von wenigstens 10 ppm zuzufügen, damit das Trennmittel nicht oxidiert werden und seine Wirksamkeit bei Reduzierung der Klebrigkeit somit erhalten bleiben soll.

Die Patentschrift US 5,066,714 mit der Bezeichnung "Curable organopolysiloxane putty-like composition" unterscheidet das Verhalten der mit internen Trennmitteln versehenen Puttymasse vor und nach dem Vernetzen. Während die aliphatischen Kohlenwasserstoffe vor der Polymerisation durch beabsichtigtes Ausschwitzen dafür sorgen, dass die Bestandteile der Puttymasse nicht an den Händen des Anwenders der Masse kleben, ein Umstand, der eine positive Eigenschaft der Masse darstellt, da durch diese Eigenschaft die praktische Handhabung der Masse vereinfacht wird, so ist dennoch die Zugabe der Trennmittel zur Zusammensetzung der dentalen Masse nachteilig, da die Freigabe der Kohlenwasserstoffe auch nach der Härtung allmählich weiterläuft und durch die Abgabe der Mittel die genaue Größe des ausgehärteten Abdrucks verfälscht wird.

In der Patentschrift wird daher vorgeschlagen, als Trennmittel anstelle aliphatischer Kohlenwasserstoffe ein Organopolysiloxan, das 5 bis 50 mol% an Alkylgruppen mit 7 bis 30 Kohlenstoffatomen aufweist, in einer Menge von 5 bis 60 Gewichtsprozent der Puttymasse, zuzufügen. Eine solche Modifikation der Masse soll dazu führen, dass das alkylgruppensubstituierte Polysiloxan in seiner Funktion als Trennmittel durch Freisetzung aus den Pasten ein klebefreies Vermischen der Komponenten mit den Händen erlaubt, wobei der Rest des Trennmittels - im Gegensatz zu den vormals eingesetzten aliphatischen Kohlenwasserstoffen - nach der Vernetzung der Puttymasse im Formstoff verbleibt.

In der WO 99/62461 mit dem Titel "Very high viscosity polysiloxane impression material" werden Putty-Abdruckmaterialien vorgeschlagen, die einen emulgierenden Weichmacher aufweisen. Bevorzugt ist der Weichmacher ein Alkylphthalat und ganz bevorzugt Octylbenzylphthalat. Die Menge des Weichmachers beträgt bevorzugt 4 - 6 Gew.-% bezogen auf die Gesamtzusammensetzung. Der Weichmacher soll die Handhabungseigenschaften des Materials verbessern und insbesondere die Klebrigkeit der Puttymasse während des Handknetens der Komponenten reduzieren. Der Weichmacher soll durch die Gegenwart eines Netzmittels emulgiert werden. Durch diesen Prozess soll verhindert werden, dass der Weichmacher freigesetzt wird und aus der Polysiloxanmatrix ausschwitzt. Beispiele 7 und 8 des Dokuments sollen zeigen, dass Octylbenzylphthalat keine Tröpfchen auf der Oberfläche des Abdruckmaterials bildet und sich so gegenüber dem Stand der Technik abhebt.

In der US 6,552,104 B1, betitelt "Method of making hydrophilic non-sweating polymerizable dental impression material" und vom gleichen Anmelder wie die WO 99/62461, wird das in der WO beschriebene Prinzip eines "im Tensid emulgierten Weichmachers", bei dem beide Substanzen eine homogene Phase bilden, weiter ausgeführt und erläutert.

Bekanntlich resultiert das Ausschwitzen des inerten Weichmachers aus der Migration der Substanz aus dem Formstoff an seine Oberfläche, wo sich der Stoff in Form flüssiger, öliger Tröpfchen abscheidet. Die Substanzwanderung aus dem Netzwerk heraus kann dazu führen, dass sich das Material eines Abdrucklöffels aus Polystyrol an- bzw. auflöst und - wie oben bereits geschildert - Abformgenauigkeit verloren geht.

In der US-Druckschrift wird definiert, dass der Begriff "Ausschwitzen" Tröpfchenbildung des Weichmachers auf der äußeren Oberfläche des gehärteten elastomeren Abdruckmaterials nach 24 Stunden Lagerung bei 23°C bedeutet.

Der Begriff "Nicht-Ausschwitzen" bedeutet, dass das elastomere Abformmaterial keine sichtbaren Tröpfchen (und/oder einen Film) des Weichmachers an seiner äußeren Oberfläche im ausgehärteten Zustand nach 24 Stunden Lagerung bei 23°C aufweist.

Vorzugsweise bedeutet der Begriff "Nicht-Ausschwitzen", dass das elastomere Abformmaterial weniger als 10 Gew.-% des Weichmachers aus der ursprünglichen Puttyzusammensetzung in Form sichtbarer Tröpfchen (und/oder eines Film) an seiner äußeren Oberfläche im ausgehärteten Zustand nach 24 Stunden Lagerung bei 23°C aufweist.

Besonders bevorzugt scheiden sich weniger als 5 Gew.-% des Weichmachers aus der ursprünglichen Puttyzusammensetzung an der Oberfläche ab und ganz besonders bevorzugt sind es weniger als 1 Gew.-%.

Der Begriff "geringer flüssiger Massenverlust" bedeutet, dass das elastomere Abformmaterial weniger als 0,05 Gew.-% Verlust nach 12 Tagen Lagerung bei 23°C erfährt.

In diesem Dokument wird das Phänomen des Ausschwitzens auch erstmals quantitativ an ausgehärteten Proben untersucht.

Als nachteilig an diesem Erfindungsprinzip muss der Einsatz der Alkylphthalate angesehen werden, da viele Verbindungen dieser chemischen Familie Probleme hinsichtlich ihrer Toxizität aufweisen und Zusammensetzungen, die Phthalate enthalten, mittlerweile speziell gekennzeichnet werden müssen.

Die Druckschrift WO 02/078647 A1 (Dentale Abformmassen auf Silikonbasis) betrifft ebenfalls Puttymaterialien auf der Basis additionsvernetzender Silikone, die im gemischten Zustand eine nicht-klebrige Konsistenz aufweisen und für eine maschinelle Mischung in üblichen motorbetriebenen Mischgeräten geeignet sind. Eine maschinelle Ausbringung und Mischung der Pasten stellt einen wichtigen technischen Fortschritt bei der Anwendung dentaler Puttymassen dar, da diese aufgrund ihrer hohen Viskosität weder mit den gängigen Spateln auf Mischblöcken verarbeitbar sind, noch eine Anwendung in einer Doppelkammerkartusche mit statischem Mischrohr möglich ist.

In dem Dokument wird vorgeschlagen, der Puttymasse als Weichmacher 4 - 10 Gew.-% mindestens eines Paraffinöls oder mindestens eines weißen Mineralöls oder einer Mischung aus mindestens einem Paraffinöl und mindestens einem weißen Mineralöl zuzufügen. Wie oben beschrieben, verleihen die Weichmacher den Pasten offensichtlich nicht nur eine "Nicht-Klebrigkeit", sondern auch eine bestimmte Geschmeidigkeit in ihrem Rheologieverhalten, die erst ein maschinelles Mischen dieser standfesten und knetbaren Massen ermöglicht.

Die WO2008/059468 beschreibt eine lager- und formstabile dentale, zweikomponentige, elastomere Abformmasse auf der Basis additionsvernetzender Silikone. Die Abformmasse enthält ein Polydimethylsiloxan umfassend ungesättigte, vernetzbare Gruppen (Vinyl). Ferner ein Polyalkylsiloxan mit Si-H-Funktionalitäten. Weiterhin einen Katalysator für die Reaktion eines Polydimethylsiloxans, umfassend vernetzbare Gruppen, mit einem Polydimethylsiloxan, umfassend Si-H-Funktionalitäten. Ferner sind ein oder mehrere Füllstoffe enthalten. Schließlich umfasst die Abformmasse als Trennmittel ein Paraffin und ein methylgestopptes Polydimethylsiloxan. Die Abformmasse weist im gemischten Zustand eine Konsistenz von 21 mm nach ISO 4823 auf. Das methylgestoppte Polydimehtylsiloxan weist ausschließlich Methylgruppen auf, d.h. Gruppen mit nur einem einzigen C-Atom.

Angesichts dieses Standes der Technik war es primäre Aufgabe der Erfindung eine lager- und formstabile dentale, zweikomponentige, elastomere Abformmasse hoher Viskosität auf der Basis additionsvernetzender Silikone anzugeben, wobei die Abformmasse im gesamten Verlauf ihres Lebenszyklus, ausgehend von der Herstellung der beiden Pasten, der Lagerphase der Pasten über den Verlauf ihrer Aushärtung bis zum vernetzten Polymerisat, weder klebrige Eigenschaften noch eine Freisetzung, bzw. ein Ausschwitzen von Substanzen aufweist. Mit dieser primären Aufgabe zur Angabe dentaler Zusammensetzungen zur Herstellung der erfindungsgemäßen dentalen Massen waren weitere Aufgaben verknüpft, entsprechende erfindungsgemäße Abdrücke anzugeben, entsprechende Kits zur Herstellung der erfindungsgemäßen Abdrücke vorzuschlagen sowie Verfahren zur Anwendung der erfindungsgemäßen Massen mitzuteilen. Gemäß dem erfinderischen Eigenschaftsprofil sollte die Puttymasse dann sowohl durch händisches Verkneten als auch maschinell mit einem üblichen motorbetriebenen automatischen Mischgerät anwendbar sein.

Die Aufgabe wird durch die Abformmasse gemäß Anspruch 1 gelöst. Ferner wird sie durch den Abdruck von Anspruch 13, die Verwendungen der Ansprüche 14 bis 16, das dentale Kit von Anspruch 17 und das Verfahren von Anspruch 18 gelöst. Vorteilhafte Ausgestaltungen der Erfindung sind in den Unteransprüchen angegeben.

Das erfindungsgemäße Basismaterial umfasst die Gruppe der additionsvernetzenden Silikone. Als kalthärtende (d.h. bei Raumtemperatur härtende) und selbsthärtende Systeme sind sie besonders gut zur Herstellung der erfindungsgemäßen Puttymassen geeignet, da sie eine sehr geringe Schrumpfung beim Härten aufweisen. Die Härtung ist hierbei eine katalysierte Additionsreaktion, die durch Hydrosilylierung erfolgt, eine Reaktion von Organohydrogenpolysiloxanen (Polysiloxane, die zum einen organische Gruppen und zum anderen Si-H Bindungen aufweisen) an Polysiloxanen, umfassend mehratomige vernetzbare Gruppen, in der Regel ungesättigte vernetzbare Gruppen und vorzugsweise (gegebenenfalls substituierte) Alkenylgruppen, insbesondere Vinyl- oder Allylgruppen.

Die erfindungsgemäßen Putty Abformmassen auf der Basis additionsvernetzender Silikone werden als Zweikomponentensysteme formuliert und bestehen aus einer sogenannten Basis- und einer Katalysatorpaste. Die Basispaste enthält die Polysiloxane, umfassend mehratomige vernetzbare Gruppen, die Organohydrogenpolysiloxane, den Füllstoff, die Kombination an Trennmitteln sowie Additive. Die Katalysatorpaste umfasst einen zweiten Anteil der Polysiloxane, umfassend mehratomige vernetzbare Gruppen, den Katalysator für die Vernetzung, ebenfalls Füllstoff sowie weitere Additive.

Bei der Umsetzung werden Si-C Bindungen geknüpft, wobei eine Si-H Bindung an eine C-C Doppelbindung addiert wird. Die Chemie der Härtung läuft somit über die direkte Addition einer SiH Funktion des Vernetzers an die ungesättigte Funktion des Polymers unter Bildung einer entsprechenden Brücke, beispielsweise einer Ethylenbrücke. Da ein solcher Mechanismus keine Abgangsgruppe oder Nebenprodukt vorsieht, haben additionsvernetzende Silikonelastomere keinen nennenswerten, durch die Polymerisation verursachten Schrumpf.

Zu den Erfindungsmerkmalen a.) bis e.) ist folgendes zu bemerken:

### Zu a.)

Es ist aus Gründen der Elastizität bevorzugt, dass eines oder mehrere der Siloxane, umfassend gegebenenfalls substituierte Alkenylgruppen, linear (d.h. im Siloxangrundgerüst unverzweigt) ist/sind. Bei einem solchen linearen Siloxan sind pro Molekül zwei der (gegebenenfalls substituierten) Alkenylgruppen, insbesondere Vinyl- oder Allylgruppen, an den Kettenenden (terminal) angeordnet.

Optional befinden sich zusätzliche solcher Gruppen zur Erhöhung des Vernetzungsgrades inmitten der Kette (d.h. nicht terminal), bevorzugt jedoch eine nicht zu hohe Anzahl, etwa pro Molekül ein oder zwei oder überhaupt keine zusätzlichen solcher Gruppen inmitten der Kette, da durch sie das Material seine elastischen Eigenschaften verliert und steifer und spröder wird.

Optional ist ebenfalls der Einsatz von verzweigten Siloxanen umfassend gegebenenfalls substituierte Alkenylgruppen.

Optional ist ebenfalls, zusätzlich zu linearen Siloxanen, sogenannte VQM-Siloxane (vinylterminierte quartär modifizierte Siloxane) einzusetzen. Es ist vorteilhaft, wenn diese aus Molekülen mit je einem Siliziumatom bestehen, das mit vier Siloxanketten substituiert ist, an deren Ende sich jeweils eine Vinylgruppe befindet. Solche Siloxane führen zu einem Gewinn an Härte des vernetzten Materials, ohne dass ein großer Flexibilitätsverlust oder eine große Sprödigkeit in Kauf genommen werden muss und stellen somit gegebenenfalls eine Alternative zu Füllstoffen dar, die in manchen Fällen die Sprödigkeit erhöhen.

Ganz besonders bevorzugt ist der Einsatz von linearen Vinylmethylpolysiloxanen. Diese weisen terminal Dimethylvinylsiloxaneinheiten auf. Weiterhin ganz besonders bevorzugt ist der Einsatz mehrerer unterschiedlich beschaffener Polysiloxane, insbesondere von linearen Vinylmethylpolysiloxanen, die unterschiedliche Viskositäten aufweisen. So ist eine Mischung zweier linearer Vinylmethylpolysiloyane ganz besonders bevorzugt, wobei beispielsweise ein lineares Vinylmethylpolysiloxan umfassend terminale Vinylgruppen eine Viskosität im Bereich von 1.000 mPas bis 20.000 mPas und ein weiteres lineares Vinylmethylpolysiloxan umfassend terminale Vinylgruppen eine Viskosität im Bereich von 55.000 mPas bis 120.000 mPas aufweist. Die Viskositätsangaben der Bestandteile beziehen sich auf die dynamischen Viskositäten, die gemäß DIN 53018 bei 25°C gemessen werden.

Die Menge der linearen Vinylmethylpolysiloxane bezogen auf die Gesamtmenge des Puttys beträgt 1 - 40 Gew.-%, bevorzugt 5 - 30 Gew.-% und besonders bevorzugt 10 - 25 Gew.-%, wobei das Gewichtsverhältnis des niedrigviskosen zum höher viskosen Siloxan im Bereich von 5:1 bis 1:5, bevorzugt 3:1 bis 1:3 und besonders bevorzugt 1:1 bis 1:3 beträgt.

### Zu b.)

Die Organohydrogenpolysiloxane sind Alkylhydrogenpolysiloxane (Polysiloxane, die zum einen Alkylgruppen und zum anderen Si-H Bindungen aufweisen), wobei die Alkylgruppen bevorzugt jeweils 1 bis 4 C-Atome tragen und besonders bevorzugt Methylgruppen sind. Organohydrogenpolysiloxane mit mindestens 3 Si-H Bindungen pro Molekül werden als Vernetzer verwendet. Zusätzlich können auch Organohydrogenpolysiloxane mit 2 Si-H Bindungen pro Molekül als sogenannte Kettenverlängerer zur Beeinflussung des Aushärteverhaltens und des Elastizitätsverhaltens eingesetzt werden.

Alternativ zum Einsatz der VQM-Siloxane kann auch von der Seite der Alkylhydrogenpolysiloxane ein Gewinn an Härte ohne großen Flexibilisierungsverlust des Polymerisats eingestellt werden. So kann zur Vernetzung beispielsweise linearer α, ω-divinylterminierter Polydimethylsiloxane das Tetrakis(dimethylsiloxy)silan als vierfachfunktioneller Vernetzer eingesetzt werden. Dieser Vernetzer ist, im Gegensatz zu den üblicherweise verwendeten Alkylhydrogenpolysiloxanen, die in Form oligomerer Gemische vorliegen, ein definiertes Molekül.

Vorzugsweise weisen die Organohydrogenpolysiloxane pro Molekül zwei bis drei Si-H Bindungen auf. Bevorzugt sind hierbei terminale Bindungen, sie können jedoch auch zusätzlich oder ausschließlich inmitten der Kette vorliegen. Organohydrogenpolysiloxane, deren Si-H Bindungen inmitten der Kette liegen, sind vorzugsweise vergleichsweise kurz.

Ganz besonders bevorzugt ist der Einsatz von Organohydrogenpolysiloxanen mit drei Si-H Bindungen pro Molekül.

Die Menge an Organohydrogenpolysiloxan wird stöchiometrisch zur Menge der Polysiloxane, umfassend mehratomige vernetzbare Gruppen, berechnet. Sie beträgt im Allgemeinen 0,5 - 10 Gew.-%, bevorzugt 0,5 - 6 Gew.-% und besonders bevorzugt 0,5 - 2,5 Gew.-% bezogen auf die Gesamtzusammensetzung des Puttys.

### Zu c.)

Die Katalyse für die Reaktion eines Polydimethysiloxans, umfassend mehratomig vernetzbare Gruppen, vorzugsweise ungesättigte vernetzbare Gruppen, mit einem Polydimethylsiloxan, umfassend Si-H Funktionalitäten, verläuft unter Beteiligung von Metallkomplexen, wobei die Si-H Gruppe sowohl an C-C Doppelbindungen und C-C Dreifachbindungen als auch an Heteroatom-Mehrfachbindungen addieren kann. Geeignete Katalysatoren sind Pt, Pd, Rh, Ni, Os oder Co. In bevorzugter Form werden die Metalle komplexiert eingesetzt. Besonders bevorzugt wird als Katalysator das Platin als Pt(0) Komplex mit Vinyl-Siloxan Liganden verwendet. Beispielhaft sei der Karstedt-Katalysator als besonders bevorzugter Katalysator genannt, der bei der Reaktion von Divinyltetramethyldisiloxan mit Hexachlorplatinsäure durch Reduktion und Komplexierung des Platins gebildet wird. Der Karstedt-Katalysator ist ein Pt(0)-Komplex, der sowohl brückenbildende als auch chelatisierende Divinyl-Liganden aufweist.

Andere geeignete und bevorzugt eingesetzte Platin-Siloxan-Komplexe, die die Additionsvernetzung beschleunigen, sind beispielsweise in den Dokumenten US 3,715,334, US 3,775,352 und US 3,814,730 beschrieben.

Der Platinkatalysator wird in Mengen im Bereich von 0,0002 bis 0,04 Gew.-%, vorzugsweise im Bereich von 10 bis 100 Gew.-ppm und besonders bevorzugt im Bereich von 15 bis 50 Gew.-ppm, jeweils berechnet als elementares Platin, bezogen auf das Gesamtgewicht aller Bestandteile der dentalen Masse, eingesetzt.

In manchen Fällen ist es zweckmäßig, mehrere Katalysatoren zu verwenden.

### Zu d.)

Die erfindungsgemäße Puttymasse enthält weiterhin Füllstoffe, da ungefüllte Silikone nach der Härtung oft noch zu elastisch sind und für die eine Anwendung als Abformmaterial noch ungeeignete Eigenschaften aufweisen. Der Einsatz der Füllstoffe erfolgt, um die angestrebten physikalischen Voraussetzungen einzustellen und zu optimieren.

Die bevorzugten Füllstoffe sind Füllstoffe wie beispielsweise Cristobalit, Silikate, Montmorillonite, Bentonite, Metalloxidpulver, wie Aluminium- oder Zinkoxide sowie deren Mischoxide, Titandioxid, Magnesiumoxid, Gips, anorganische Salze wie Sulfate, Carbonate sowie Glas. Weiterhin bevorzugt ist kristallines Siliziumdioxid, wie pulverisierter Quarz oder Diatomeenerde. Zu den bevorzugten Füllstoffen zählen auch nanoskalige Kieselsäuren, die in Form nicht-aggregierter und nicht-agglomerierter Partikel vorliegen und beispielsweise nach dem Sol-Gel-Verfahren herstellbar sind.

Die Füllstoffe können oberflächenbehandelt und dabei bevorzugt hydrophobiert sein, beispielsweise durch die Behandlung ihrer Oberflächen mit Organosilanen. Die Füllstoffe werden so gewählt, dass eine Shore A Härte, bestimmt nach DIN 53505, des vernetzten Abdrucks von 45 bis 85, bevorzugt im Bereich von 60 bis 80, resultiert.

Der Füllstoff kann in der einen und/oder der anderen Komponente der zweikomponentigen Masse untergebracht sein. Bevorzugt wird er in ähnlichen Mengen beiden Komponenten beigegeben.

Im Allgemeinen werden die Füllstoffe in Mengen von 50 - 90 Gew.-%, bevorzugt von 55 - 80 Gew.-% und besonders bevorzugt von 60 - 70 Gew.-% bezogen auf das Gesamtgewicht der Puttymasse eingesetzt.

### Zu e.)

Erfindungsgemäß ist ferner der Einsatz von Trennmitteln bzw. Weichmachern in der Zusammensetzung der Puttymasse. Die Begriffe Weichmacher und Trennmittel werden synonym verwendet. Die Kombination aus Paraffin und alkylsubstituierten Polydimethylsiloxanen in dentalen Puttymassen ist neu. Es wurde überraschenderweise gefunden, dass bei Zugabe einer Kombination dieser Substanzen eine Puttymasse formuliert werden kann, die im gesamten Verlauf ihres Lebenszyklus, ausgehend von der Herstellung der beiden Pasten, der Lagerphase der Pasten über den Verlauf ihrer Aushärtung bis zum vernetzten Polymerisat, weder klebrige Eigenschaften noch eine Freisetzung, bzw. ein Ausschwitzen von Substanzen aufweist. Die Mischbarkeit der klebfreien, knetbaren und standfesten Pasten ist so gut, dass ein maschinelles Mischen ermöglicht ist.

Paraffin bezeichnet ein Gemisch aus linearen und verzweigten gesättigten Kohlenwassersstoffen.

Bevorzugte Paraffine sind Mischungen von flüssigen und festen Kohlenwasserstoffen. Die feste Phase kann dabei aus kristallinen und mikrokristallinen Komponenten bestehen. Bevorzugt ist das Paraffin eine Mischung aus 60 bis 70% einer flüssigen Phase aus n- und iso-Paraffin und einer festen Phase bestehend aus kristallinen Komponenten (10 bis 20% n-Paraffine) und mikrokristallinen Komponenten (isoParaffine). Ganz besonders bevorzugt ist das Paraffin Vaseline (auch "Petrolatum" genannt).

Das in der vorliegenden Erfindung zur Verwendung geeignete Petrolatum umfasst jeden Grad von weißem oder gelbem Petrolatum. Im Allgemeinen kann jeder Viskositäts- oder Konsistenzgrad von Petrolatum, der im Stand der Technik bekannt ist, in dieser Erfindung verwendet werden. Die vorliegende Erfindung bezieht Varianten ein, bei denen teilweise Petrolatum durch Gemische von Kohlenwasserstoffmaterialien ersetzt ist, die formuliert werden können, um Petrolatum in Aussehen und der Konsistenz nachzuahmen. Zum Beispiel kann solch eine Kombination durch Schmelzen von Mineralöl in verschiedenen Anteilen mit Substanzen, wie z.B. mikrokristallinem Wachs, Paraffinwachs und ähnlichem gebildet werden.

Bevorzugte alkylsubstituierte Polydimethylsiloxane tragen in ihrer Alkylkette wenigstens 8, bevorzugt wenigstens 16 und besonders bevorzugt wenigstens 18 C-Atome. Die Alkylkette kann linear oder verzweigt sein. Alternativ können auch Polydimethylsiloxane eingesetzt werden, bei denen die lineare oder verzweigte Alkylkette vollständig oder teilweise halogeniert, bevorzugt fluoriert, ist. Die Länge der Seitenkette beträgt maximal 30 C-Atome, bevorzugt maximal 23 C-Atome.

Es wird eine Kombination aus 2 - 8 Gew.-%, bevorzugt 2 - 5 Gew.-% und besonders bevorzugt 2 - 4 Gew.-% mindestens eines alkylsubstituierten Polydimethylsiloxans mit 1 - 9 Gew.-%, bevorzugt 2 - 8 Gew.-% und besonders bevorzugt 3 - 7 Gew.-% an Paraffin, bezogen auf das Gesamtgewicht der Puttymasse an Trennmitteln eingesetzt.

Bei vorteilhafter Ausgestaltung der Erfindung kommen zu den Merkmalen a.) bis e.) die Merkmale f.) und/oder g.) hinzu.

### Zu f.)

Eine besonders bevorzugte Ausgestaltung der erfindungsgemäßen Abformmasse weist zusätzliche Bestandteile gemäß Merkmal f) auf.

Die Reaktion zwischen den beiden Komponenten findet bei Umgebungstemperatur (insbesondere im Mund des Patienten) statt und ist innerhalb weniger Minuten abgeschlossen. Es ist daher in vielen Fällen notwendig, einen Reaktionsverzögerer, der auch Inhibitor genannt wird, einzusetzen, um die Reaktion zu kontrollieren. Im Allgemeinen umfasst die Verzögererkomponente jegliche ungesättigte Substanzen mit niedrigem Molekulargewicht, die zu Beginn in der Polymerisation verbraucht werden, um ein Härten zu verzögern. Derartige Inhibitoren sind beispielsweise in den Druckschriften US 3,933,880, US 3,445,420 und US 3,989,667 beschrieben. Beispiele hierfür sind acetylenisch ungesättigte Alkohole wie 3-Methyl-1-butin-3-ol, 1-Ethinylcyclohexan-1-ol oder 3-Methyl-1-pentin-1-ol. Sie können einzeln oder zusammen verwendet werden. Es können auch Verbindungen auf Vinylsiloxanbasis wie 1,1,3,3-Tetramethyl-1,3-divinyldisiloxan und/oder vinylgruppenhaltige Oligo- und Disiloxane benutzt werden.

Weitere Typen von Verzögerern sind Verbindungen, die zwei oder mehr Doppelbindungen aufweisen und vorzugsweise durch Abstraktion eines Allyl-H-Atoms ein delokalisiertes Elektonensystem ausbilden können, das sich über 5 C-Atome erstreckt. Solche Verbindungen können aus der Gruppe bestehend aus α-Terpinenen, β-Terpinenen, γ-Terpinenen, α-Phellandren, β-Phellandren und Terpinolen ausgewählt werden. Bevorzugt ausgewählt sind Substanzen, die über eine exo-Doppelbindung verfügen.

Die bevorzugt eingesetzte Menge der Inhibitorkomponente wird in erster Linie vom Typ des eingesetzten Inhibitors bestimmt, so dass keine allgemeingültigen Bereiche angegeben werden können. Wird beispielsweise das 1,3-Divinyltetramethyldisiloxan verwendet, so müssen wenigstens 0,05 bis 0,15 Gew.-%, bezogen auf die Gesamtmasse, eingesetzt werden. Wird auf einen ethinylisch ungesättigten Alkohol zurückgegriffen, so reichen 0,0001 - 0,001 Gew.-%, bevorzugt 0,0005 - 0,007 Gew.-% und besonders bevorzugt 0,0009 - 0,002 Gew.-% bezogen auf das Gesamtgewicht der Puttymasse.

Besonders bevorzugt verwendete Inhibitoren sind daher ethylinisch ungesättigte Alkohole.

Im Laufe der Vernetzung des Polysiloxansystems kann es zu einer Freisetzung von Wasserstoff kommen. Aus diesem Grund wird den erfindungsgemäßen Zusammensetzungen ein Metall zugesetzt, das vorzugsweise fein verteilt vorliegt. Bevorzugt wird als Metall Platin oder Palladium eingesetzt. Das Metall kann auch auf einem Salz abgesondert werden. Das Platin wird in einer Menge von 1 bis 1000 ppm, bevorzugt 1 bis 500 ppm und besonders bevorzugt 10 bis 50 ppm eingesetzt.

Als Stabilisatoren werden ebenfalls wasserabsorbierende anorganische Feststoffe wie wasserfreies Calciumsulfat, Calciumchlorid oder ähnliche Verbindungen oder wasseradsorbierende Verbindungen wie Zeolithe, Molekularsiebe oder ähnliche Substanzen der erfindungsgemäßen Zusammensetzung beigegeben. Die Menge der wasseraufnehmenden Substanz liegt zwischen 0,1 bis 5 Gew.-%, bevorzugt zwischen 0,25 - 4 Gew.-% und besonders bevorzugt zwischen 0,5 - 2 Gew.-%. Der Stabilisator wird nicht d.) zugerechnet.

Durch den gezielten Einsatz von Kunststoffpulvern und/oder ultrahochmolekularen Siloxanen als Rheologiemodifizierer können die rheologischen Eigenschaften der Puttymasse so gezielt eingestellt werden, dass die Wirkungen der o.g. Trennmittel weithin unterstützt werden und somit eine maschinelle Mischung und Austragbarkeit des Materials während der Anwendung erleichtert wird. In eigenen Untersuchungen hat sich gezeigt, dass Materialien ohne die hier unten genannten nicht-verstärkend wirkenden Füllstoffe sich zwar gut maschinell austragen lassen, durch den Einsatz derselbigen jedoch eine höhere als die übliche Ausdruckgeschwindigkeit möglich ist. Auch dieser Befund war überraschend. Die Menge der Rheologiemodifizierer liegt im Bereich zwischen 1 bis 50 Gew.-%, bevorzugt 1 bis 20 Gew.-% und besonders bevorzugt 1 bis 10 Gew.-%. Neben organischen Polymerisaten und hoch- bis ultrahochmolekularen Siloxanen, die in ihren Viskositäten im Grenzbereich zwischen hochviskos und bereits fest anzusiedeln sind, können auch pyrogene Kieselsäuren bzw. Kieselguren eingesetzt werden. Die Rheologiemodifizierer, auch wenn es sich dabei um feste Materialien handelt, werden nicht Merkmal d.) von Anspruch 1 zugerechnet.

Zu den Rheologiemodifizierern zählen auch Feststoffe aus Silikonharzen, die im Silikonpolymer löslich sind.

### Zu g.)

Gemäß einer weiteren Ausgestaltung weist die erfindungsgemäße Puttymasse weitere, zusätzliche Bestandteile gemäß Merkmal g) auf.

Demnach kann die erfindungsgemäße Putty-Zusammensetzung Farbstoffe wie Pigmente, Netzmittel (oberflächenaktive Mittel) wie Tenside, antioxidationsmittel sowie medizinische und/oder arzneiliche Wirkstoffe enthalten.

Basis- und Katalysatorpaste liegen in einem Volumenverhältnis in einem Bereich von 1:1 bis 10:1 vor.

Die erfindungsgemäßen Massen können mit den Händen gemischt werden. Bevorzugt liegen beide Pasten dann in einem Volumenverhältnis von 1:1 vor. Bei der Handmischvariante wird die Masse mit Dosierlöffeln aus ihren Lagerbehältnissen (beispielsweise Plastikdosen) genommen, per Hand vermischt und im angemischten Zustand in einen Abformlöffel gegeben. Mit dem Löffel wird ein Abdruck der betreffenden dentalen Situation im Mund des Patienten entweder nach dem Verfahren der Korrekturabformung oder nach dem Verfahren der Doppelmischabformung genommen. Nach der Vernetzung und dem Herausnehmen des Abdrucks aus dem Patientenmund kann der Abdruck dann noch beschnitten, getrimmt oder anderweitig bearbeitet werden.

Die erfindungsgemäßen Massen können auch mit üblichen motorbetriebenen automatischen Mischgeräten ausgebracht und verwendet werden. In solchen Fällen liegen Basis- und Katalysatorpaste in einem Volumenverhältnis in einem Bereich von 1:1 bis 10:1, bevorzugt in einem Bereich von 4:1 bis 6:1 und besonders bevorzugt in einem Bereich von 5:1 vor.

Das neue lager- und formstabile dentale, zweikomponentige, elastomere Puttymaterial hoher Viskosität auf der Basis additionsvernetzender Silikone, wobei eine Komponente einen Polymerisationskatalysator umfasst, enthält in der Gesamtzusammensetzung somit
a.) mindestens ein Polydimethylsiloxan umfassend mehratomige vernetzbare Gruppen, vorzugsweise ungesättigte vernetzbare Gruppen,
b.) mindestens ein Polyalkylsiloxan mit Si-H Funktionalitäten,
c.) mindestens einen Katalysator für die Reaktion eines Polydimethysiloxans, umfassend mehratomig vernetzbare Gruppen, mit einem Polydimethylsiloxan, umfassend Si-H Funktionalitäten,
d.) einen oder mehrere Füllstoffe,
e.) eine Kombination aus Trennmitteln, umfassend
   i. mindestens ein alkylsubstituiertes Polydimethylsiloxan bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
   ii. mindestens ein Paraffin,
f.) optional mindestens ein oder mehrere zusätzliche Bestandteile, wie
   i. Reaktionsverzögerer,
   ii. Wasserabsorptionsmittel, die nicht Bestandteil von d.) sind
   iii. Wasserstoffabsorptionsmittel,
   iv. Rheologiemodifizierer, die nicht Bestandteil von d.) sind sowie
g.) optional weitere Additive wie Netzmittel und/oder Antioxidationsmittel und/oder Farbstoffe und/oder medizinische und/oder arzneiliche Wirkstoffe.

Eine bevorzugte erfindungsgemäße Gesamtzusammensetzung enthält somit
a.) mindestens ein Polydimethylsiloxan umfassend (gegebenenfalls substituierte) Alkenylgruppen, insbesondere Vinylgruppen oder Allylgruppen,
b.) mindestens ein Organohydrogenpolysiloxan mit zwei bis drei Si-H Bindungen pro Molekül,
c.) mindestens einen Platin Katalysator für die Reaktion eines Polydimethysiloxans, umfassend Vinylgruppen, mit einem Polydimethylsiloxan, umfassend Si-H Funktionalitäten,
d.) einen oder mehrere Füllstoffe
e.) eine Kombination aus Trennmitteln, umfassend
   i. mindestens ein alkylsubstituiertes Polydimethylsiloxan bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
   ii. mindestens ein Paraffin,
f.) optional zusätzliche Bestandteile, wie
   i. mindestens einen Reaktionsverzögerer ausgewählt aus der Gruppe von Cycloalkanolen und kurzkettigen Siloxanen, wobei beide Gruppen ungesättigte Seitenketten tragen, und/oder
   ii. mindestens ein Wasserabsorptionsmittel wie Zeolith oder wasserbindende Salze, die nicht d.) zugerechnet werden und/oder
   iii. mindestens ein wasserstoffabsorbierendes Metall und/oder
   iv. mindestens einen Rheologiemodifizierer aus der Gruppe von Kunststoffpulvern, ultrahochmolekularen Siloxanen sowie pyrogenen Kieselsäuren, mit der Maßgabe, dass die Rheologiemodifizierer nicht d.) zugerechnet werden sowie
g.) optional weitere Additive wie Netzmittel und/oder Antioxidationsmittel und/oder Farbstoffe und/oder medizinische und/oder arzneiliche Wirkstoffe.

Eine besonders bevorzugte erfindungsgemäße Gesamtzusammensetzung enthält somit
a.) mindestens ein lineares Polysiloxan umfassend terminale Vinylgruppen mit einer Viskosität im Bereich von 1.000 mPas bis 20.000 mPas und mindestens ein weiteres lineares Polysiloxan umfassend terminale Vinylgruppen mit einer Viskosität im Bereich von 55.000 mPas bis 120.000 mPas,
b.) mindestens ein Organohydrogenpolysiloxan mit mindestens drei Si-H Bindungen,
c.) mindestens einen Karstedt Katalysator für die Reaktion eines Polydimethysiloxans, umfassend Vinylgruppen, mit einem Polydimethylsiloxan, umfassend Si-H Funktionalitäten,
d.) einen oder mehrere Füllstoffe
e.) eine Kombination aus Trennmitteln, umfassend
   i. mindestens ein alkylsubstituiertes Polydimethylsiloxan bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
   ii. mindestens ein Paraffin in Form von Vaseline,
f.) zusätzliche Bestandteile, wie
   i. mindestens Ethinylcyclohexanol als Reaktionsverzögerer und/oder
   ii. mindestens ein Zeolith als wasserabsorbierendes Mittel, das nicht d.) zugerechnet wird und/oder
   iii. mindestens fein verteiltes, nicht komplexiertes Platin als wasserstoffabsorbierendes Metall und/oder
   iv. mindestens Kunstoffpulver und ultrahochmolekulares Siloxan als Rheologiemodifizierer, mit der Maßgabe, dass die Rheologiemodifizierer nicht d.) zugerechnet werden sowie
g.) optional weitere Additive wie Netzmittel und/oder Antioxidationsmittel und/oder Farbstoffe und/oder medizinische und/oder arzneiliche Wirkstoffe.

Eine ganz besonders bevorzugte erfindungsgemäße Gesamtzusammensetzung enthält somit
a.) 1 - 40 Gew.-%, bevorzugt 5 - 30 Gew.-%, besonders bevorzugt 10 - 25 Gew.-% mindestens ein lineares Polysiloxan umfassend terminale Vinylgruppen mit einer Viskosität im Bereich von 1.000 mPas bis 20.000 mPas und mindestens ein weiteres lineares Polysiloxan umfassend terminale Vinylgruppen mit einer Viskosität im Bereich von 55.000 mPas bis 120.000 mPas,
b.) 0,5 - 10 Gew.-%, bevorzugt 0,5 - 6 Gew.-%, besonders bevorzugt 0,5 - 2,5 Gew.-% mindestens ein Organohydrogenpolysiloxan mit mindestens drei Si-H Bindungen,
c.) 0,0002 bis 0,04 Gew.-%, vorzugsweise 0,001 bis 0,01 Gew.-% und besonders bevorzugt0,0015 bis 0,005 Gew.-%, mindestens einen Karstedt Katalysator für die Reaktion eines Polydimethysiloxans, umfassend Vinylgruppen, mit einem Polydimethylsiloxan, umfassend Si-H Funktionalitäten,
d.) 50 - 90 Gew.-%, bevorzugt 55 - 80 Gew.-% und besonders bevorzugt 60 - 70 Gew.-% eines oder mehrerer Füllstoffe
e.) eine Kombination aus Trennmitteln, umfassend
   i. 2 - 8 Gew.-%, bevorzugt 2 - 5 Gew.-% und besonders bevorzugt 2 - 4 Gew.-% mindestens eines alkylsubstituierten Polydimethylsiloxans bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
   ii. 1 - 9 Gew.-%, bevorzugt 2 - 8 Gew.-% und besonders bevorzugt 3 - 7 Gew.-% mindestens eines flüssigen Paraffins in Form von Vaseline
f.) zusätzliche Bestandteile, wie
   i. 0,0001 - 0,001 Gew.-%, bevorzugt 0,0005 - 0,007 Gew.-% und besonders bevorzugt 0,0009 - 0,002 Gew.-% mindestens eines Ethinylcyclohexanol als Reaktionsverzögerer, und/oder
   ii. 0,1 bis 5 Gew.-%, bevorzugt 0,25 - 4 Gew.-% und besonders bevorzugt 0,5 - 2 Gew.-% mindestens eines Zeoliths als wasserabsorbierendes Mittel, das nicht d.) zugerechnet wird, und/oder
   iii.1 bis 1000 ppm, bevorzugt 1 bis 500 ppm und besonders bevorzugt 10 bis 50 ppm mindestens eines fein verteilten, nicht komplexierten Platins als wasserstoffabsorbierendes Metall, und/oder
   iv. 1 bis 50 Gew.-%, bevorzugt 1 bis 20 Gew.-% und besonders bevorzugt 1 bis 10 Gew.-% mindestens eines Kunstoffpulvers und eines ultrahochmolekularen Siloxans als Rheologiemodifizierer, mit der Maßgabe, dass die Rheologiemodifizierer nicht d.) zugerechnet werden sowie
g.) optional weitere Additive wie Netzmittel und/oder Antioxidationsmittel und/oder Farbstoffe und/oder medizinische und/oder arzneiliche Wirkstoffe.

Es war überraschend, dass dentale Zusammensetzungen, wie oben angegeben, sämtliche Anforderungen an eine Putty-Masse erfüllen, wie sie im Rahmen der vorliegenden Erfindung angegeben werden sollen.

Die vorliegende Erfindung umfasst auch Abdrücke, die aus Anwendungen der erfindungsgemäßen Zusammensetzungen hergestellt werden sowie Verfahren zur Herstellung erfindungsgemäßer Abdrücke.

Gemäß einem weiteren Aspekt betrifft die vorliegende Erfindung auch Kits zur Herstellung der erfindungsgemäßen Abdrücke, umfassend oder bestehend aus erfindungsgemäßen Zusammensetzungen sowie Verwendungen der erfindungsgemäßen Abformmassen.

Ein Ausführungsbeispiel der erfindungsgemäßen Abformmasse (Rezeptur A) wurde mit einer Abformmasse gemäß dem Stand der Technik (Rezeptur B) verglichen.

Nachfolgend sind die Rezepturen angegeben und die darin enthaltenden Komponenten erläutert.

Rezepturen Mischungsbeispiele (Anteile in Gewichtsprozent)

### A Basis

| | |
|---|---|
| Si-H funktionaliertes Polydimethylsiloxan (Si-H Gehalt: 4,2 mmol/g; Viskosität: 40 mPas) | 1,50 |
| Vinyl-funktionalisiertes Polydimethylsiloxan (Vinylgehalt: 0,05 mmol/g; Viskosität: 10000 mPas) | 5,7975 |
| Vinyl-funktionalisiertes Polydimethylsiloxan (Vinylgehalt: 0,03 mmol/g; Viskosität: 65000 mPas) | 11,90 |
| 1-Ethinylcyclohexanol | 0,0025 |
| Stearyl Dimethicone | 2,90 |
| Ultrahochmolekulares Polydimethylsiloxan (Viskosität: 1000000 mPas) | 3,70 |
| Vaseline | 4,50 |
| Polytetrafluorethylenpulver | 1,00 |
| Na-A Zeolith (Porenweite ∼ 4Å) | 1,00 |
| Cristobalitmehl (silanisiert) | 66,70 |
| Farbpigmente | 1,00 |
| | |
| Summe: | 100,00 |

### A Katalysator

| | |
|---|---|
| Karstedt Katalysator | 0,004 |
| Vinyl-funktionalisiertes Polydimethylsiloxan (Vinylgehalt: 0,05 mmol/g; Viskosität: 10000 mPas) | 7,396 |
| Vinyl-funktionalisiertes Polydimethylsiloxan (Vinylgehalt: 0,03 mmol/g; Viskosität: 65000 mPas) | 11,80 |
| Vinyl-funktionalisiertes Polydimethylsiloxan (Vinylgehalt: 1,4 mmol/g; Viskosität: 2200 mPas) | 0,80 |
| Stearyl Dimethicone | 2,90 |
| Ultrahochmolekulares Polydimethylsiloxan (Viskosität: 1000000 mPas) | 3,50 |
| Vaseline | 4,90 |
| Polytetrafluorethylenpulver | 1,00 |
| Na-A Zeolith (Porenweite ∼ 4Å) | 1,00 |
| Cristobalitmehl (silanisiert) | 66,70 |
| | |
| Summe: | 100,00 |

### B Katalysator

| | |
|---|---|
| Vinyl-funktionalisiertes Polydimethylsiloxan (Vinylgehalt: 0,03 mmol/g; Viskosität: 65000 mPas) | 14,97 |
| Vinyl-funktionalisiertes Polydimethylsiloxan (Vinylgehalt: 0,05 mmol/g; Viskosität: 10000 mPas) | 6,999 |
| Kieselgur | 1,81 |
| Cristobalitmehl (silanisiert) | 70,19 |
| flüssiges Paraffinöl (Viskosität: 160 mPas) | 6,02 |
| Karstedt Katalysator | 0,0035 |
| fein verteiltes Platin | 0,0075 |
| | |
| Summe: | 100,00 |

### B Basis

| | |
|---|---|
| Vinyl-funktionalisiertes Polydimethylsiloxan (Vinylgehalt: 0,03 mmol/g; Viskosität: 65000 mPas) | 13,93 |
| Si-H funktionalisiertes Polydimethylsiloxan (Si-H-Gehalt: 4,7 mmol/g; Viskosität: 40 mPas) | 1,12 |
| Vinyl-funktionalisiertes Polydimethylsiloxan (Vinylgehalt: 0,13 mmol/g; Viskosität: 1000 mPas) | 5,39 |
| Kieselgur | 1,95 |
| flüssiges Paraffinöl (Viskosität: 160 mPas) | 6,18 |
| Cristobalitmehl (silanisiert) | 70,30 |
| Si-H funktionalisiertes Polydimethylsiloxan (Si-H-Gehalt: 3,4 mmol/g; Viskosität: 3 mPas) | 0,53 |
| Farbpigmente | 0,60 |
| | |
| Summe: | 100,00 |

Die Pasten der Beispiele A und B wurden durch inniges Vermischen der Komponenten in einem Hauschildmischer hergestellt. Anschließend wurden die Materialien für 10 min im Vakuum entlüftet.

Die gemäß ISO4823 gemessenen Konsistenzen betragen:
Vergleichsbeispiel A: 28mm
Vergleichsbeispiel B: 23,5 mm

Demnach erfüllen beide Materialien das erforderliche Kriterium (<35 mm) zur Einstufung als Abformmaterial mit einer knetbaren Putty-Konsistenz (Typ 0).

Um das oben beschriebene "Nicht-Ausschwitzen" der Abformmaterialien zu beurteilen wurden im Einzelnen folgende Testungen durchgeführt. Zum einen wurde subjektiv geprüft, ob sich auf der Oberfläche der Einzelpasten oder dem ausgehärteten Material Flüssigkeitstropfen erkennen lassen. Hierbei konnte für Material A kein Ausschwitzen beobachtet werden, wohingegen sich auf der Oberfläche der Einzelpasten von Beispiel B Flüssigkeitstropfen zeigten. Zum anderen wurde das "Nicht-Ausschwitzen" bzw. der "geringe flüssige Massenverlust" quantifiziert, indem die Einzelpasten und das ausgehärtete Material in definierten zeitlichen Abständen mit einem Zellstofftuch abgewischt wurden und jeweils zuvor und anschließend gewogen wurden. Eventuelle Abscheidungen werden in diesem Falle durch das Tuch entfernt und führen somit zu einem messbaren Massenverlust.

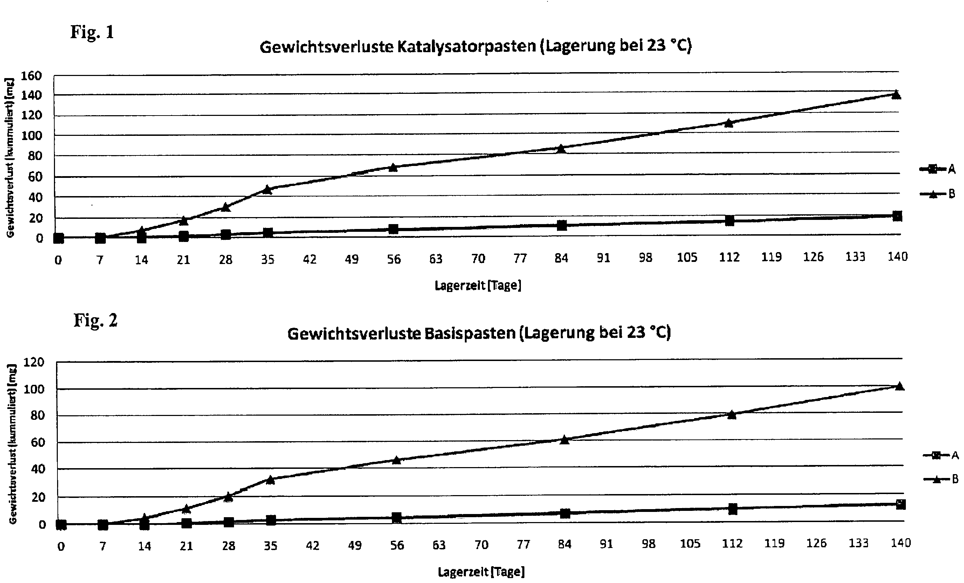

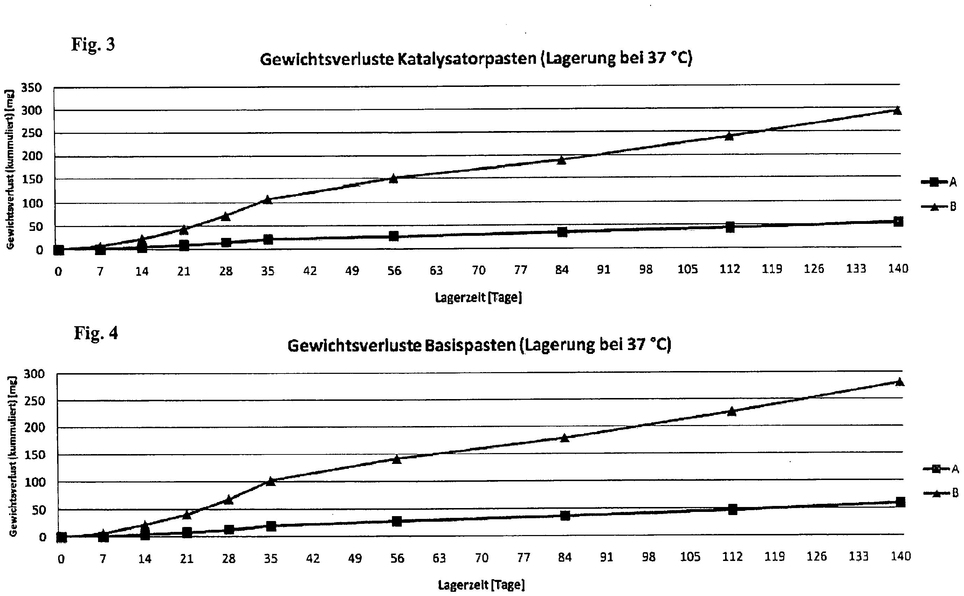

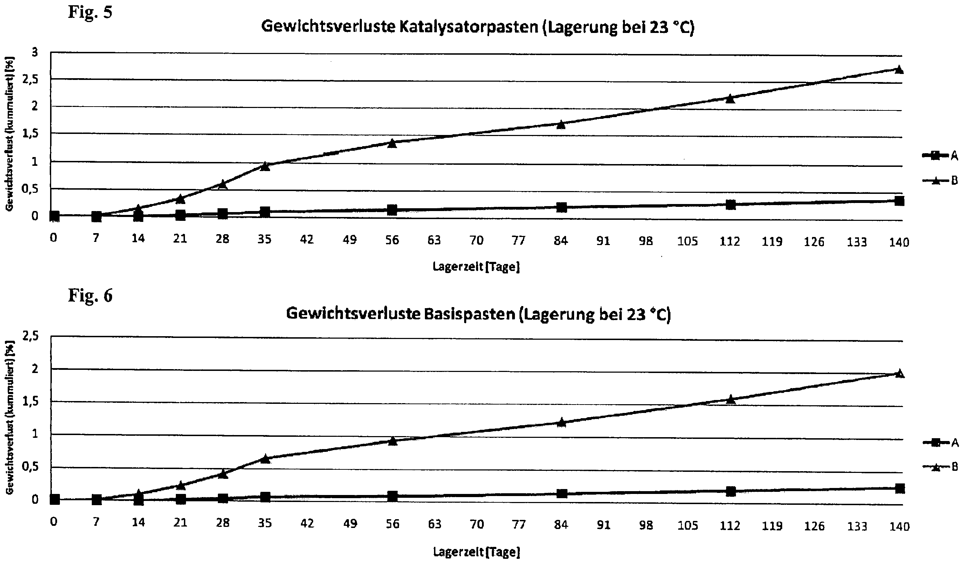

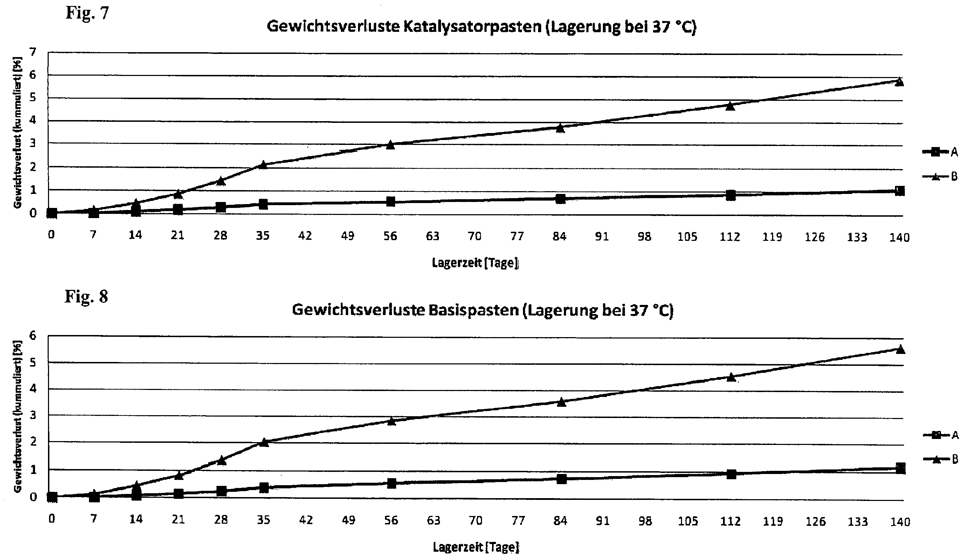

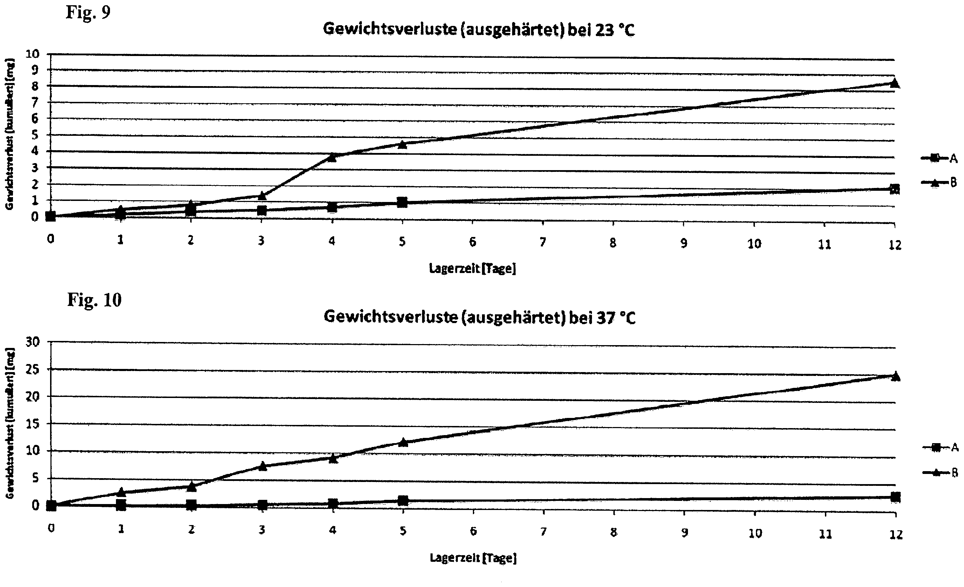

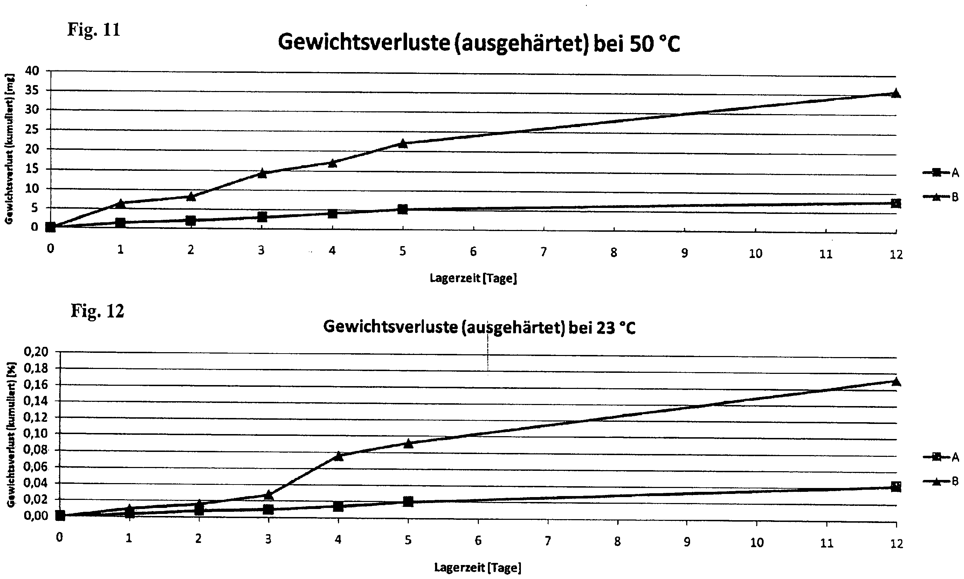

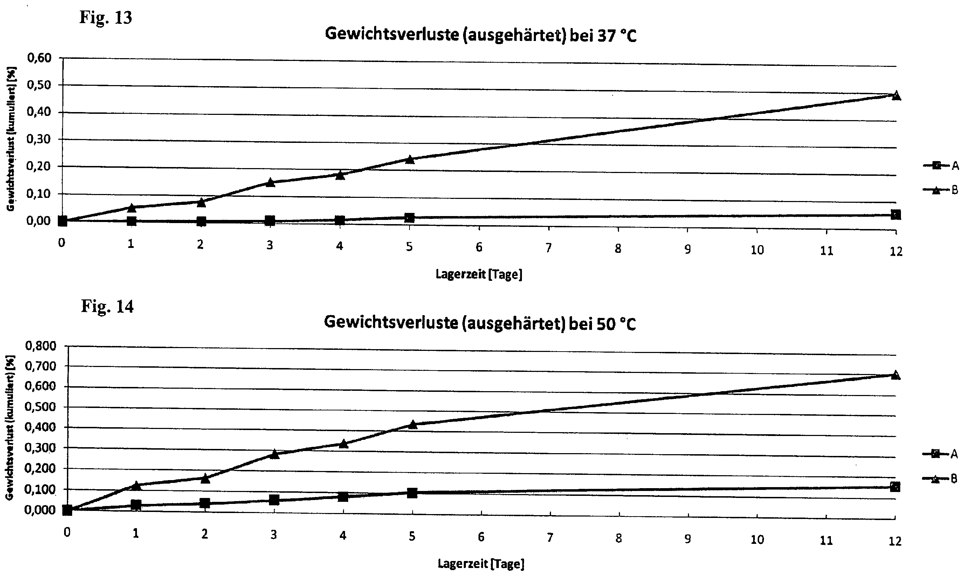

In den Figuren 1 bis 14 sind die Messergebnisse für zwei verschiedene Stabilitätsuntersuchungen aufgeführt. Die Fig. 1 bis 8 (Fig. 1 bis 4 mit absolut Werten in mg und Fig. 5 bis 8 in Gew.%) zeigen die durch das Ausschwitzen verursachten kumulierten Gewichtsverluste der Einzelpasten bei 23 bzw. 37 °C innerhalb von 140 Tagen. Die Fig. 9 bis 14 zeigen dann die Gewichtsverluste für die bereits ausgehärteten Silikonformulierungen bei 23, 37 und 50 °C, ebenfalls in absoluten mg (Fig. 9 bis 11) und in Prozent (Fig. 12 bis 14). Diese sind für die ersten fünf Tage gezeigt. Für die Messungen des Ausschwitzverhaltens wurde wie folgt vorgegangen:
Eine etwa 5 g schwere Probe des jeweiligen Materials (für die ausgehärteten Beispiele wurden je 2,5 g Basis- und Katalysatorpaste durch Kneten mit der Hand innig vermischt und anschließend für 15 min bei Raumtemperatur ausgehärtet) wurde auf eine Acetatfolie gelegt und exakt gewogen.

Die Pasten aus den Beispielen A und B lassen sich gut mit den Händen kneten und kleben dabei nicht. Diese "Nicht-Klebrigkeit" ist, wie bereits oben beschrieben, besonders vorteilhaft für die Anwendung als Putty-Material".

Anschließend wurden die Proben in einer Plastikdose bei 23 °C bzw. 37 °C gelagert. Nach dem jeweils angegebenen Zeitraum wurde mit einem Zellstofftuch Flüssigkeit, die sich auf der Oberfläche gesammelt hatte, abgetupft und anschließend das Gewicht erneut bestimmt.

Legt man die Definition aus der US 6,552,104 B1 zu Grunde, bedeutet das "Nicht-Ausschwitzen" einen Massenverlust der Weichmacher von weniger als 1 Gew.% innerhalb von 24 Stunden und ein "geringer flüssiger Massenverlust" eine Abnahme des Gewichtes der ausgehärteten Probekörper von weniger als 0,05 Gew. % nach 12 Tagen bei 23 °C. Das erfindungsgemäße Beispiel A weist nach dieser Zeit bei 23°C einen Massenverlust von 0,04 Gew.% auf.

Der besondere Vorteil der Verwendung des alkylsubstituierten Polydimethylsiloxans wird deutlich, wenn man beachtet, dass Rezeptur A 1,5 Gew.% mehr Weichmacher enthält als Rezeptur B und dennoch einen geringen, durch das Ausschwitzen während der Lagerung bedingten, Gewichtsverlust.

## Patentansprüche

1. Lager- und formstabile dentale, zweikomponentige, elastomere Abformmasse auf der Basis additionsvernetzender Silikone, wobei eine Komponente mindestens einen Katalysator umfasst, enthaltend oder bestehend aus
a.) mindestens ein Polydimethylsiloxan umfassend mehratomige vernetzbare Gruppen, vorzugsweise ungesättigte vernetzbare Gruppen,
b.) mindestens ein Polyalkylsiloxan mit Si-H Funktionalitäten,
c.) den Katalysator für die Reaktion eines Polydimethylsiloxans, umfassend mehratomig vernetzbare Gruppen, mit einem Polydimethylsiloxan, umfassend Si-H Funktionalitäten,
d.) einen oder mehrere Füllstoffe,
e.) eine Kombination aus Trennmitteln, umfassend
i.) mindestens ein alkylsubstituiertes Polydimethylsiloxan, bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
ii.) mindestens ein Paraffin,
wobei die Masse im gemischten Zustand eine nach ISO 4823 bestimmte Konsistenz von kleiner oder gleich 35 mm aufweist.

2. Lager- und formstabile dentale, zweikomponentige, elastomere Abformmasse nach Anspruch 1, die
a.) mindestens ein Polydimethylsiloxan umfassend (gegebenenfalls substituierte) Alkenylgruppen, insbesondere Vinylgruppen oder Allylgruppen,
b.) mindestens ein Organohydrogenpolysiloxan mit zwei bis drei Si-H Bindungen pro Molekül,
c.) mindestens einen Platin Katalysator für die Reaktion eines Polydimethylsiloxans, umfassend Vinylgruppen, mit einem Polydimethylsiloxan, umfassend Si-H Funktionalitäten,
d.) einen oder mehrere Füllstoffe
e.) eine Kombination aus Trennmitteln, umfassend
i.) mindestens ein alkylsubstituiertes Polydimethylsiloxan, bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
ii.) mindestens ein Paraffin
enthält.

3. Lager- und formstabile dentale, zweikomponentige, elastomere Abformmasse nach einem der Ansprüche 1 und 2, die
a.) mindestens ein lineares Polysiloxan umfassend terminale Vinylgruppen mit einer Viskosität im Bereich von 1.000 mPas bis 20.000 mPas und mindestens ein weiteres lineares Polysiloxan umfassend terminale Vinylgruppen mit einer Viskosität im Bereich von 55.000 mPas bis 120.000 mPas,
b.) mindestens ein Organohydrogenpolysiloxan mit mindestens drei Si-H Bindungen,
c.) mindestens einen Karstedt Katalysator für die Reaktion eines Polydimethylsiloxans, umfassend Vinylgruppen, mit einem Polydimethylsiloxan, umfassend Si-H Funktionalitäten,
d.) einen oder mehrere Füllstoffe
e.) eine Kombination aus Trennmitteln, umfassend
i.) mindestens ein alkylsubstituiertes Polydimethylsiloxan, bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
ii.) mindestens ein Paraffin in Form von Vaseline
enthält.

4. Lager- und formstabile dentale, zweikomponentige, elastomere Abformmasse nach einem der vorstehenden Ansprüche, die
a.) 1 - 40 Gew.-%, bevorzugt 5 - 30 Gew.-%, besonders bevorzugt 10 - 25 Gew.-% mindestens ein lineares Polysiloxan umfassend terminale Vinylgruppen mit einer Viskosität im Bereich von 1.000 mPas bis 20.000 mPas und mindestens ein weiteres lineares Polysiloxan umfassend terminale Vinylgruppen mit einer Viskosität im Bereich von 55.000 mPas bis 120.000 mPas, wobei das Gewichtsverhältnis des niedrigviskosen zum höher viskosen Siloxan im Bereich von 5:1 bis 1:5, bevorzugt 3:1 bis 1:3 und besonders bevorzugt 1:1 bis 1:3 beträgt.
b.) 0,5 - 10 Gew.-%, bevorzugt 0,5 - 6 Gew.-%, besonders bevorzugt 0,5 - 2,5 Gew.-% mindestens ein Organohydrogenpolysiloxan mit mindestens drei Si-H Bindungen,
c.) 0,0002 bis 0,04 Gew.-%, vorzugsweise 0,001 bis 0,01 Gew.-% und besonders bevorzugt 0,0015 bis 0,005 Gew.-%, mindestens einen Karstedt Katalysator für die Reaktion eines Polydimethylsiloxans, umfassend Vinylgruppen, mit einem Polydimethylsiloxan, umfassend Si-H Funktionalitäten,
d.) 50 - 90 Gew.-%, bevorzugt 55 - 80 Gew.-% und besonders bevorzugt 60 - 70 Gew.-% eines oder mehrerer Füllstoffe
e.) eine Kombination aus Trennmitteln, umfassend
i.) 2 - 8 Gew.-%, bevorzugt 2 - 5 Gew.-% und besonders bevorzugt 2 - 4 Gew.-% mindestens eines alkylsubstituierten Polydimethylsiloxans, bei dem die Alkylkette linear oder verzweigt ist, und/oder mindestens ein alkylsubstituiertes Polydimethylsiloxan, bei dem die Alkylkette linear oder verzweigt sowie teilweise oder vollständig halogeniert, bevorzugt fluoriert ist, wobei das alkylsubstituierte Polydimethylsiloxan in seiner Alkylkette, die verzweigt oder linear sein kann, wenigstens 8, bevorzugt wenigstens 16 und besonders bevorzugt wenigstens 18 C-Atome trägt und maximal 30 C-Atome, bevorzugt maximal 23 C-Atome trägt, und
ii.) 1 - 9 Gew.-%, bevorzugt 2 - 8 Gew.-% und besonders bevorzugt 3 - 7 Gew.-% mindestens eines Paraffins in Form von Vaseline
enthält, wobei die Gewichtsangaben auf die Masse der Gesamtzusammensetzung bezogen sind.

5. Lager- und formstabile dentale, zweikomponentige, elastomere Abformmasse nach einem der Ansprüche 1 bis 4, wobei die Alkylgruppe des alkylsubstituierten Polydimethylsiloxans linear ist.

6. Lager- und formstabile dentale, zweikomponentige, elastomere Abformmasse nach einem der vorstehenden Ansprüche, wobei der Füllstoff ausgewählt ist aus der Gruppe von Cristobalit, Silikaten, Montmorilloniten, Bentoniten, Metalloxidpulvern, wie Aluminium- oder Zinkoxide sowie deren Mischoxide, Titandioxid, Magnesiumoxid, Gips, anorganischen Salzen wie Sulfate, Carbonate sowie Glas, kristallines Siliziumdioxid oder Diatomeenerde sowie nanoskalige Kieselsäuren, die in Form nicht-aggregierter und nicht-agglomerierter Partikel vorliegen.

7. Lager- und formstabile dentale, zweikomponentige, elastomere Abformmasse nach Anspruch 6, wobei der Füllstoff Cristobalit umfasst.

8. Lager- und formstabile dentale, zweikomponentige, elastomere Abformmasse nach einem der vorstehenden Ansprüche, umfassend eine Basis- und eine Katalysatorpatse, wobei das Mischungsverhältnis im Volumen der Basis- zur Katalysatorpaste in der Handmischvariante 1: 1 und in der maschinenbetriebenen Variante im Bereich von 1:1 bis 10:1, bevorzugt im Bereich von 4:1 bis 6:1 und besonders bevorzugt 5:1 beträgt.

9. Lager- und formstabile dentale, zweikomponentige, elastomere Abformmasse nach einem der Ansprüche 1 bis 8, enthaltend
f.) mindestens ein oder mehrere zusätzliche Bestandteile, wie
i. Reaktionsverzögerer und/oder
ii. Wasserabsorptionsmittel, die nicht Bestandteil von d.) sind und/oder
iii. Wasserstoffabsorptionsmittel und/oder
iv. Rheologiemodifizierer, die nicht Bestandteil von Merkmal d.) sind und/oder
g.) weitere Additive wie Netzmittel und/oder Antioxidationsmittel und/oder Farbstoffe und/oder medizinische und/oder arzneiliche Wirkstoffe.

10. Lager- und formstabile Abformmasse nach einem der Ansprüche 1 bis 9, enthaltend
f.) zusätzliche Bestandteile, wie
i. mindestens einen Reaktionsverzögerer ausgewählt aus der Gruppe von Cycloalkanolen und kurzkettigen Siloxanen, wobei in beiden Fällen die Verbindungen ungesättigte Seitenketten tragen, und/oder
ii. mindestens ein Wasserabsorptionsmittel wie Zeolith, Molekularsieb oder wasserbindende Salze, die nicht Merkmal d.) zugerechnet werden, und/oder
iii. mindestens ein wasserstoffabsorbierendes Metall, und/oder
iv. mindestens einen Rheologiemodifizierer aus der Gruppe von Kunststoffpulvern, ultrahochmolekularen Siloxanen sowie pyrogenen Kieselsäuren und Kieselguren, mit der Maßgabe, dass die Rheologiemodifizierer nicht d.) zugerechnet werden und/oder
g.) weitere Additive wie Netzmittel und/oder Antioxidationsmittel und/oder Farbstoffe und/oder medizinische und/oder arzneiliche Wirkstoffe.

11. Lager- und formstabile Abformmasse nach einem der Ansprüche 1 bis 10, enthaltend
f.) zusätzliche Bestandteile, wie
i. mindestens Ethinylcyclohexanol als Reaktionsverzögerer und/oder
ii. mindestens ein Zeolith als wasserabsorbierendes Mittel, das nicht d.) zugerechnet wird und/oder
iii. mindestens fein verteiltes, nicht komplexiertes Platin als wasserstoffabsorbierendes Metall und/oder
iv. mindestens Kunststoffpulver und/oder ultrahochmolekulares Siloxan und/oder Kieselgur(en) als Rheologiemodifizierer, mit der Maßgabe, dass die Rheologiemodifizierer nicht Merkmal d.) zugerechnet werden und/oder
g.) weitere Additive wie Netzmittel und/oder Antioxidationsmittel und/oder Farbstoffe und/oder medizinische und/oder arzneiliche Wirkstoffe.

12. Lager- und formstabile Abformmasse nach einem der Ansprüche 1 bis 11, enthaltend
f.) zusätzliche Bestandteile, wie
i. 0,0001 - 0,001 Gew.-%, bevorzugt 0,0005 - 0,007 Gew.-% und besonders bevorzugt 0,0009 - 0,002 Gew.-% Ethinylcyclohexanol als Reaktionsverzögerer, und/oder
ii. 0,1 bis 5 Gew.-%, bevorzugt 0,25 - 4 Gew.-% und besonders bevorzugt 0,5 - 2 Gew.-% mindestens eines Zeoliths als wasserabsorbierendes Mittel, das nicht d.) zugerechnet wird, und/oder
iii. 1 bis 1000 ppm, bevorzugt 1 bis 500 ppm und besonders bevorzugt 10 bis 50 ppm mindestens eines fein verteilten, nicht komplexierten Platins als wasserstoffabsorbierendes Metall, und/oder
iv. 1 bis 50 Gew.-%, bevorzugt 1 bis 20 Gew.-% und besonders bevorzugt 1 bis 10 Gew.-% mindestens eines Kunststoffpulvers und eines ultrahochmolekularen Siloxans als Rheologiemodifizierer, mit der Maßgabe, dass die Rheologiemodifizierer nicht Merkmal d.) zugerechnet werden und/oder
g.) weitere Additive wie Netzmittel und/oder Antioxidationsmittel und/oder Farbstoffe und/oder medizinische und/oder arzneiliche Wirkstoffe,
wobei die Gewichtsangaben auf die Masse der Gesamtzusammensetzung bezogen sind.

13. Dentaler Abdruck, hergestellt durch Vernetzung einer lager- und formstabilen dentalen, zweikomponentigen, elastomeren Abformmasse nach einem der Ansprüche 1 bis 12.

14. Verwendung einer lager- und formstabilen dentalen, zweikomponentigen, elastomeren Abformmasse nach einem der Ansprüche 1 bis 12 aus Kartuschen in maschinenbetriebenen Automischgeräten.

15. Verwendung einer lager- und formstabilen dentalen, zweikomponentigen, elastomeren Abformmasse nach einem der Ansprüche 1 bis 12 beim Kneten von Hand.

16. Verwendung einer lager- und formstabilen dentalen, zweikomponentigen, elastomeren Abformmasse nach einem der Ansprüche 1 bis 12 in der Korrekturabformung oder in der Doppelmischabformung.

17. Dentales Kit, umfassend entweder
• eine lager- und formstabile dentale, zweikomponentige, elastomere Abformmasse nach einem der Ansprüche 1 bis 12,
• eine oder mehrere Mischkanülen,
• optional Abformlöffel oder optional Material zur Herstellung eines individuellen Abformlöffels,
• optional ein leicht-, mittel- oder schwerfließendes Korrekturabformmaterial,
oder
• eine lager- und formstabile dentale, zweikomponentige, elastomere Abformmasse nach einem der Ansprüche 1 bis 12,
• einen oder mehrere Dosierlöffel,
• optional Abformlöffel oder optional Material zur Herstellung eines individuellen Abformlöffels,
• optional ein leicht-, mittel- oder schwerfließendes Korrekturabformmaterial,
• optional einen Anmischblock.

18. Verfahren zur Herstellung eines dentalen Abdrucks, umfassend die Schritte
• Bereitstellen einer lager- und formstabilen dentalen, zweikomponentigen, elastomeren Abformmasse nach einem der Ansprüche 1 bis 12,
• Bereitstellen einer geeigneten Form,
• Befüllen der Form mit der angemischten lager- und formstabilen dentalen, zweikomponentigen, elastomeren Abformmasse,
• Positionieren der befüllten Form im Patientenmund,
• Aushärtenlassen der angemischten lager- und formstabilen dentalen, zweikomponentigen, elastomeren Abformmasse,
• Herausnehmen des erhaltenen Abdrucks aus dem Patientenmund,
• gegebenenfalls Beschneiden, Trimmen oder anderweitiges Bearbeiten des erhaltenen Abdrucks.

## Claims

1. A two-component dental elastomeric impression material with dimensional stability and storage durability based on addition curing silicones, wherein one component comprises at least one catalyst, containing:
a.) at least one polydimethylsiloxane comprising polyatomic cross-linkable groups, preferably unsaturated cross-linkable groups,
b.) at least one polyaklysiloxane with Si-H functionalities,
c.) the catalyst for the reaction of a polydimethysiloxane, comprising multiple atomic cross-linkable groups, with a polydimethylsiloxane, comprising Si-H functionalities,
d.) one or more fillers,
e.) a combination of release agents, comprising
i.) at least an alkyl substituted polydimethylsiloxane, in which the alkyl chain is linear or branched, and/or at least an alkyl substituted polydimethylsiloxane, in which the alkyl chain is linear or branched, and partially or completely halogenated, preferably fluorinated, wherein the alkyl-substituted polydimethylsiloxane in the alkyl chain thereof, which can be linear or branched, has at least 8, preferably at least 16 and particularly preferably at least 18 C-atoms, and has a maximum of 30 carbon atoms, preferably a maximum of 23 C-Atoms, and
ii.) at least one paraffin;
wherein the mass in the mixed state has a consistency of less than or equal to 35 mm, determined according to ISO 4823.

2. The two-component dental elastomeric impression material with dimensional stability and storage durability according to claim 1, which containing
a.) at least one polydimethylsiloxane comprising (possibly substituted) alkenyl groups, in particular vinyl groups or allyl groups,
b.) at least one organohydrogenpolysiloxane with two to three Si-H bonds per molecule,
c.) at least one platinum catalyst for the reaction of a polydimethylsiloxane, comprising vinyl groups, with a polydimethylsiloxane comprising Si-H functionalities,
d.) one or more fillers,
e.) a combination of release agents, comprising
i.) at least one alkyl-substituted polydimethylsiloxane, in which the alkyl chain is linear or branched, and/or at least one alkyl-substituted polydimethylsiloxane, in which the alkyl chain is linear or branched, and partially or completely halogenated, preferably fluorinated, wherein the alkyl-substituted polydimethylsiloxane in the alkyl chain thereof, which can be linear or branched, has at least 8, preferably at least 16 and particularly preferably at least 18 C-atoms, and has a maximum of 30 carbon atoms, preferably a maximum of 23 C-Atoms, and
ii.) at least one paraffin.

3. The two-component dental elastomeric impression material with dimensional stability and storage durability according to claim 1 or 2, containing
a.) at least one linear polysiloxane comprising terminal vinyl groups with a viscosity in the range of 1,000 mPas to 20,000 mPas, and at least one further linear polysiloxane comprising terminal vinyl groups with a viscosity in the range of 55,000 mPas to 120,000 mPas,
b.) at least one organohydrogen polysiloxane with at least three Si--H bonds,
c.) at least one Karstedt catalyst for the reaction of a polydimethysiloxane, comprising vinyl groups, with a polydimethylsiloxane comprising Si--H functionalities,
d.) one or more fillers,
e.) a combination of release agents, comprising
i.) at least one alkyl-substituted polydimethylsiloxane, in which the alkyl chain is linear or branched, and/or at least one alkyl-substituted polydimethylsiloxane, in which the alkyl chain is linear or branched, and partially or completely halogenated, preferably fluorinated, wherein the alkyl-substituted polydimethylsiloxane in the alkyl chain thereof, which can be linear or branched, has at least 8, preferably at least 16 and particularly preferably at least 18 C-atoms, and has a maximum of 30 carbon atoms, preferably a maximum of 23 C-Atoms, and
ii.) at least one paraffin in the form of Vaseline.

4. The two-component dental elastomeric impression material with dimensional stability and storage durability according to one of the preceding claims, containing
a.) 1-40%, preferably 5-30%, particularly preferable 10-25%, by weight of at least one linear polysiloxane comprising terminal vinyl groups with a viscosity in the range of 1,000 mPas to 20,000 mPas, and at least one further linear polysiloxane comprising terminal vinyl groups with a viscosity in the range of 55,000 mPas to 120,000 mPas, wherein the weight ratio of the low viscosity siloxanes to the higher viscosity siloxanes is in the range of 5:1 to 1:5, preferably in the range of 3:1 to 1:3 and particularly preferable in the range of 1:1 to 1:3,
b.) 0.5-10%, preferably 0.5-6%, particularly preferable 0.5-2.5%, by weight of at least one organohydrogen polysiloxane with at least three Si-H bonds,
c.) 0.0002 to 0.04%, preferably 0.0001 to 0.01%, particularly preferable 0.0015 to 0.005%, by weight of at least one Karstedt catalyst for the reaction of a polydimethysiloxane, comprising vinyl groups with a polydimethylsiloxane comprising Si--H functionalities,
d.) 50-90%, preferably 55-80%, particularly preferable 60-70%, by weight one or more fillers,
e.) a combination of release agents, comprising
i.) 2-8%, preferably 2-5%, particularly preferable 2-4%, by weight of at least one alkyl-substituted polydimethylsiloxane, in which the alkyl chain is linear or branched, and/or at least one alkyl-substituted polydimethylsiloxane, in which the alkyl chain is linear or branched, and partially or completely halogenated, preferably fluorinated, wherein the alkyl-substituted polydimethylsiloxane in the alkyl chain thereof, which can be linear or branched, has at least 8, preferably at least 16 and particularly preferably at least 18 C-atoms, and has a maximum of 30 carbon atoms, preferably a maximum of 23 C-Atoms, and
ii.) 1-9%, preferably 2-8%, particularly preferable 3-7%, by weight of at least one paraffin in the form of Vaseline,
wherein the weight specifications are relative to the mass of the total composition.

5. The two-component dental elastomeric impression material with dimensional stability and storage durability according to one of claims 1 to 4, wherein the alkyl group of the alkyl-substituted polydimethylsiloxane is linear.

6. The two-component dental elastomeric impression material with dimensional stability and storage durability according to one of the preceding claims, wherein the filler is selected from the group of cristobalite, silicate, montmorillonite, bentonite, metal oxide powder, titanium dioxide, magnesium oxide, gypsum, inorganic salts, and glass, crystalline silicon dioxide or diatomaceous earth and nanoscaled silicic acids that are present in the form of non-aggregated and non-agglomerated particles.

7. The two-component dental elastomeric impression material with dimensional stability and storage durability according to claim 6, wherein the filler comprises cristobalite.

8. The two-component dental elastomeric impression material with dimensional stability and storage durability according to one of the preceding claims, comprising a base paste and a catalyst paste, wherein the mixture ratio in volume of base paste to catalyst paste in a hand mixable variant is 1:1, and in a machine operated variant is in the range of 1:1 to 10:1, preferably in the range of 4:1 to 6:1 and particularly preferably 5:1.

9. The two-component dental elastomeric impression material with dimensional stability and storage durability according to one of claims 1 to 8, containing
f.) at least one of the following components
i.) retarding agents and/or
ii.) water absorption substance, that is not a component of d.) and/or
iii.) hydrogen absorption substance and/or
iv.) rheology modifiers that are not a component of feature d), and/or
g.) further additives, such as wetting agents, and/or antioxidants and/or dyes, and/or medical and/or pharmaceutical active agents.

10. The impression material with dimensional stability and storage durability according to one of claims 1 to 9, containing
f.) at least one of the following components,
i.) at least one retarding agent selected from the group of cycloalkanols and short chain siloxanes, wherein in both cases, the compounds comprise unsaturated side chains, and/or
ii.) at least one water absorption substance selected from the group consisting of zeolite, molecular sieves or hydrophilic salts, that are not included in d.) and/or
iii.) at least one hydrogen absorbing metal, and/or
iv.) at least one rheology modifier selected from the group consisting of pyrogenic silicic acids and kieselguhrs, with the stipulation that the rheology modifier is not included in d), and/or
g.) further additives, such as wetting agents, and/or antioxidants and/or dyes, and/or medical and/or pharmaceutical active agents.

11. The impression material with dimensional stability and storage durability according to one of claims 1 to 10, containing
f.) at least one of the following component,
i.) at least ethinylcyclohexanol as a retarding agent and/or
ii.) at least one zeolite as a water absorbing substance that is not included in d.), and/or
iii.) at least finely distributed non-complexed platinum as a hydrogen absorbing metal, and/or
iv.) at least plastic powder and/or ultra-high molecular weight siloxane and/or kieselguhr(s) as rheology modifiers, with the stipulation that the rheology modifiers are not included in feature d), and/or
g.) further additives, such as wetting agents, and/or antioxidants and/or dyes, and/or medical and/or pharmaceutical active agents.

12. The impression material with dimensional stability and storage durability according to one of claims 1 to 11, containing
f.) at least one of the following components,
i.) 0.0001-0.001%, preferably 0.0005 to 0.007%, particularly preferable 0.0009 to 0.002%, by weight of ethinylcyclohexanol as a retarding agent, and/or
ii.) 0.1-5%, preferably 0.25-4%, particularly preferable 0.5-2%, by weight of at least one zeolite as a water absorbing substance, that is not included in d), and/or
iii.) 1 to 1000 ppm, preferably 1 to 500 ppm, particularly preferable 10 to 50 ppm, of at least a finely distributed, non-complexed platinum as a hydrogen absorbing metal, and/or
iv.) 1 to 50%, preferably 1 to 20%, particularly preferable 1 to 10%, by weight of at least one plastic powder and an ultra-high molecular weight siloxane as a rheology modifier, with the stipulation that the rheology modifier is not included in feature d), and/or
g.) further additives, such as wetting agents, and/or antioxidants and/or dyes, and/or medical and/or pharmaceutical active agents,
wherein the weight specifications are relative to the mass of the total composition.

13. A dental impression, produced by cross-linking a two-component dental elastomeric impression material with dimensional stability and storage durability according to one of claims 1 to 12.

14. Use of a two-component dental elastomeric impression material with dimensional stability and storage durability according to one of claims 1 to 12 from cartridges for machine-operated automatic mixing devices.

15. Use of a two-component dental elastomeric impression material with dimensional stability and storage durability according to one of claims 1 to 12 by kneading via hand.

16. Use of a two-component dental elastomeric impression material with dimensional stability and storage durability according to one of claims 1 to 12 in the correction impression or in the double mixing impression.

17. A dental kit comprising either
• a two-component dental elastomeric impression material with dimensional stability and storage durability according to one of claims 1 to 12,
• one or more mixing tips,
• optionally an impression tray or optionally material for producing an individual impression tray,
• optionally light, medium, or heavy flowing correction impression material,
or
• a two-component dental elastomeric impression material with dimensional stability and storage durability according to one of claims 1 to 12,
• one or more metering spoons,
• optionally an impression tray or optionally material for producing an individual impression tray,
• optionally light, medium, or heavy flowing correction impression material,
• soptionally a mixing block.

18. A method for producing a dental impression, comprising the steps of
• preparing a two-component dental elastomeric impression material with dimensional stability and storage durability according to one of claims 1 to 12,
• preparing a suitable form,
• filling the form with the mixed two-component dental elastomeric impression material with dimensional stability and storage durability,
• positioning the filled form in the patient's mouth,
• hardening the mixed two-component dental elastomeric impression material with dimensional stability and storage durability,
• removing the resulting mold from the patient's mouth,
• if applicable, cutting, trimming or otherwise processing the resulting mold.

## Revendications

1. Matériau de prise d'empreinte dentaire en élastomère à deux composants, indéformable et stable au stockage, à base de silicones réticulés par addition, dans lequel un composant comporte au moins un catalyseur, contenant ou étant constitué de :
a.) au moins un polydiméthylsiloxane comportant des groupes réticulables polyatomiques, de préférence des groupes réticulables insaturés,
b.) au moins un polydiméthylsiloxane avec des fonctionnalités Si-H,
c.) le catalyseur pour la réaction d'un polydiméthylsiloxane, comportant des groupes réticulables polyatomiques, avec un polydiméthylsiloxane, comportant des fonctionnalités Si-H,
d.) un ou plusieurs agents de charge,
e.) une combinaison d'agents de séparation, comportant
i.) au moins un polydiméthylsiloxane substitué par un alkyle dans lequel la chaîne alkyle est linéaire ou ramifiée, et/ou au moins un polydiméthylsiloxane substitué par un alkyle dans lequel la chaîne alkyle est linéaire ou ramifiée et partiellement ou complètement halogénisée, de préférence fluorée, le polydiméthylsiloxane substitué par un alkyle portant au moins 8, de préférence au moins 16 et de façon particulièrement préférentielle au moins 18 atomes C et au maximum 30 atomes C, avantageusement au maximum 23 atomes C, dans sa chaîne alkyle, laquelle peut être ramifiée ou linéaire, et
ii.) au moins une paraffine,
dans lequel la masse présente une consistance inférieure ou égale à 35 mm à l'état mélangé selon la norme ISO 4823.

2. Matériau de prise d'empreinte dentaire en élastomère à deux composants, indéformable et stable au stockage, selon la revendication 1, lequel contient :
a.) au moins un polydiméthylsiloxane présentant des groupes alcényles (éventuellement substitués), en particulier des groupes vinyles ou des groupes allyles,
b.) au moins un organohydrogénopolysiloxane avec deux à trois liaisons Si-H par molécule,
c.) au moins un catalyseur au platine pour la réaction d'un polydiméthylsiloxane, comportant des groupes vinyles, avec un polydiméthylsiloxane, comportant des fonctionnalités Si-H,
d.) un ou plusieurs agents de charge,
e.) une combinaison d'agents de séparation, comportant
i.) au moins un polydiméthylsiloxane substitué par un alkyle dans lequel la chaîne alkyle est linéaire ou ramifiée, et/ou au moins un polydiméthylsiloxane substitué par un alkyle dans lequel la chaîne alkyle est linéaire ou ramifiée et partiellement ou complètement halogénisée, de préférence fluorée, le polydiméthylsiloxane substitué par un alkyle portant au moins 8, de préférence au moins 16 et de façon particulièrement préférentielle au moins 18 atomes C et au maximum 30 atomes C, avantageusement au maximum 23 atomes C, dans sa chaîne alkyle, laquelle peut être ramifiée ou linéaire, et
ii.) au moins une paraffine.

3. Matériau de prise d'empreinte dentaire en élastomère à deux composants, indéformable et stable au stockage, selon l'une des revendications 1 et 2, lequel contient :
a.) au moins un polysiloxane linéaire comportant des groupes vinyles terminaux avec une viscosité comprise entre 1000 mPas et 20.000 mPas, et au moins un autre polysiloxane linéaire comportant des groupes vinyles terminaux avec une viscosité comprise entre 55.000 mPas et 120.000 mPas,
b.) au moins un organohydrogénopolysiloxane avec au moins trois liaisons Si-H,
c.) au moins un catalyseur de Karstedt pour la réaction d'un polydiméthylsiloxane comportant des groupes vinyles, avec un polydiméthylsiloxane comportant des fonctionnalités Si-H,
d.) un ou plusieurs agents de charge,
e.) une combinaison d'agents de séparation, comportant
i.) au moins un polydiméthylsiloxane substitué par un alkyle dans lequel la chaîne alkyle est linéaire ou ramifiée, et/ou au moins un polydiméthylsiloxane substitué par un alkyle dans lequel la chaîne alkyle est linéaire ou ramifiée et partiellement ou complètement halogénisée, de préférence fluorée, le polydiméthylsiloxane substitué par un alkyle portant au moins 8, de préférence au moins 16 et de façon particulièrement préférentielle au moins 18 atomes C et au maximum 30 atomes C, avantageusement au maximum 23 atomes C, dans sa chaîne alkyle, laquelle peut être ramifiée ou linéaire, et
ii.) au moins une paraffine sous la forme de vaseline.

4. Matériau de prise d'empreinte dentaire en élastomère à deux composants, indéformable et stable au stockage, selon l'une des revendications précédentes, lequel contient
a.) 1-40% en poids, de préférence 5-30% en poids, de façon particulièrement préférentielle 10-25% en poids d'au moins un polysiloxane linéaire comportant des groupes vinyles terminaux avec une viscosité comprise entre 1000 mPas et 20.000 mPas, et d'au moins un autre polysiloxane linéaire comportant des groupes vinyles terminaux avec une viscosité comprise entre 55.000 mPas et 120.000 mPas, où le rapport pondéral du siloxane faiblement visqueux au siloxane hautement visqueux est compris entre 5:1 et 1:5, de préférence entre 3:1 et 1:3 et de façon particulièrement préférentielle entre 1:1 et 1:3,
b.) 0,5-10% en poids, de préférence 0,5-6% en poids, de façon particulièrement préférentielle 0,5-2,5% en poids d'au moins un organohydrogénopolysiloxane avec au moins trois liaisons Si-H,
c.) de 0,0002 à 0,04% en poids, de préférence de 0,001 à 0,01% en poids et de façon particulièrement préférentielle de 0,0015 à 0,005% en poids d'au moins un catalyseur de Karstedt pour la réaction d'un polydiméthylsiloxane comportant des groupes vinyles, avec un polydiméthylsiloxane comportant des fonctionnalités Si-H,
d.) 50-90% en poids, de préférence 55-80% en poids et de façon particulièrement préférentielle 60-70% en poids d'un ou de plusieurs agents de charge,
e.) une combinaison d'agents de séparation, comportant
i.) 2-8% en poids, de préférence 2-5% en poids et de façon particulièrement préférentielle 2-4% en poids d'au moins un polydiméthylsiloxane substitué par un alkyle dans lequel la chaîne alkyle est linéaire ou ramifiée, et/ou d'au moins un polydiméthylsiloxane substitué par un alkyle dans lequel la chaîne alkyle est linéaire ou ramifiée et partiellement ou complètement halogénisée, de préférence fluorée, le polydiméthylsiloxane substitué par un alkyle portant au moins 8, de préférence au moins 16 et de façon particulièrement préférentielle au moins 18 atomes C et au maximum 30 atomes C, avantageusement au maximum 23 atomes C, dans sa chaîne alkyle, laquelle peut être ramifiée ou linéaire, et
ii.) 1-9% en poids, de préférence 2-8% en poids et de façon particulièrement préférentielle 3-7% en poids d'au moins une paraffine sous la forme de vaseline,
dans lequel les indications de poids sont rapportés à la masse de la composition globale.

5. Matériau de prise d'empreinte dentaire en élastomère à deux composants, indéformable et stable au stockage, selon l'une des revendications 1 à 4, dans lequel le groupe alkyle du polydiméthylsiloxane substitué par un alkyle est linéaire.

6. Matériau de prise d'empreinte dentaire en élastomère à deux composants, indéformable et stable au stockage, selon l'une des revendications précédentes, dans lequel l'agent de charge est sélectionné parmi le groupe comprenant du cristobalite, des silicates, des montmorillonites, des bentonites, des poudres d'oxyde de métal, tels que des oxydes d'aluminium ou de zinc ainsi que leurs oxydes mixtes, le dioxyde de titane, d'oxyde de magnésium, le gypse, des sels anorganiques tels que des sulfates, du carbonate tel que le verre, le dioxyde de silicium cristallin ou la terre de diatomées ainsi que des acides siliciques nanométriques, lesquels se présentent sous la forme de particules non agrégées et non agglomérées.

7. Matériau de prise d'empreinte dentaire en élastomère à deux composants, indéformable et stable au stockage, selon la revendication 6, dans lequel l'agent de charge comporte de la cristobalite.

8. Matériau de prise d'empreinte dentaire en élastomère à deux composants, indéformable et stable au stockage, selon l'une des revendications précédentes, comportant une pâte de base et une pâte catalytique, dans lequel le rapport de mélange dans le volume de la pâte de base à la pâte catalytique est de 1:1 dans la variante de mélange manuel et dans la plage de 1:1 à 10:1 de préférence dans la plage de 4:1 à 6:1 et de façon particulièrement préférentielle de 5:1 dans la variante réalisée à la machine.

9. Matériau de prise d'empreinte dentaire en élastomère à deux composants, indéformable et stable au stockage, selon l'une des revendications 1 à 8, contenant :
f.) au moins un ou plusieurs composants supplémentaires, tels que :
i.) des retardateurs de réaction et/ou
ii.) des matières absorbant l'eau non inclus dans d.) et/ou
iii.) des matières absorbant l'hydrogène, et/ou
iv.) des modificateurs rhéologiques non inclus dans la caractéristique d.), et/ou
g.) d'autres additifs tels que des agents mouillants et/ou des agents antioxydants et/ou des colorants et/ou des substances actives thérapeutiques et/ou médicales.

10. Matériau de prise d'empreinte indéformable et stable au stockage, selon l'une des revendications 1 à 9, contenant :
f.) des composants supplémentaires, tels que,
i.) au moins un retardateur de réaction sélectionné parmi le groupe des cycloalcanols et des siloxanes à chaîne courte, la liaison portant des chaînes latérales insaturées dans les deux cas, et/ou
ii.) au moins une matière absorbant l'eau, telle que la zéolithe, un filtre moléculaire ou des sels rétenteurs d'eau, non attribuables à la caractéristique d.), et/ou
iii.) au moins un métal absorbant l'hydrogène, et/ou
iv.) au moins un modificateur rhéologique issu du groupe des poudres synthétiques, des siloxanes de masse moléculaire très élevée tels que des acides siliciques pyrogènes et des diatomites, à condition que les modificateurs rhéologiques ne soient pas attribuables à d.), et/ou
g.) d'autres additifs tels que des agents mouillants et/ou des agents antioxydants et/ou des colorants et/ou des substances actives thérapeutiques et/ou médicales.

11. Matériau de prise d'empreinte indéformable et stable au stockage selon l'une des revendications 1 à 10, contenant :
f.) des composants supplémentaires, tels que
i.) au moins de l'éthynylcyclohexanol en tant que retardateur de réaction, et/ou
ii.) au moins un zéolithe en tant que matière absorbant l'eau, non attribuable à d.), et/ou
iii.) au moins du platine finement dispersé et non complexé, en tant que métal absorbant l'hydrogène, et/ou
iv.) au moins une poudre synthétique et/ou du siloxane de masse moléculaire très élevée et/ou un ou des diatomites en tant que modificateur(s) rhéologique(s), à condition que les modificateurs rhéologiques ne soient pas attribuables à la caractéristique d.), et/ou
g.) d'autres additifs tels que des agents mouillants et/ou des agents antioxydants et/ou des colorants, des substances actives thérapeutiques et/ou médicales.

12. Matériau de prise d'empreinte indéformable et stable au stockage selon l'une des revendications 1 à 11, contenant :
f.) des composants supplémentaires, tels que :
i.) 0,0001-0,001% en poids, de préférence 0,0005-0,007% en poids et de façon particulièrement préférentielle 0,0009-0,002% en poids d'éthynylcyclohexanol en tant que retardateur de réaction, et/ou
ii.) de 0,1 à 5% en poids, de préférence de 0,25 à 4% en poids et de façon particulièrement préférentielle de 0,5 à 2% en poids d'au moins un zéolithe en tant que matière absorbant l'eau, non-attribuable à d.), et/ou
iii.) de 1 à 1000 ppm, de préférence de 1 à 500 ppm et de façon particulièrement préférentielle de 10 à 50 ppm d'au moins un platine finement dispersé et non complexé, en tant que métal absorbant l'hydrogène, et/ou
iv.) de 1 à 50% en poids, de préférence de 1 à 20% en poids et de façon particulièrement préférentielle de 1 à 10% en poids d'au moins une poudre synthétique et d'un siloxane de masse moléculaire très élevée en tant que modificateur rhéologique, à condition que les modificateurs rhéologiques ne soient pas attribuables à la caractéristique d.), et/ou
g.) d'autres additifs tels que des agents mouillants et/ou des agents antioxydants et/ou des colorants et/ou des substances actives thérapeutiques et/ou médicales,
dans lequel les indications de poids sont rapportés à la masse de la composition globale.

13. Empreinte dentaire, fabriquée par mouillage d'un matériau de prise d'empreinte dentaire en élastomère à deux composants, indéformable et stable au stockage selon l'une des revendications 1 à 12.

14. Utilisation d'un matériau de prise d'empreinte dentaire en élastomère à deux composants, indéformable et stable au stockage selon l'une des revendications 1 à 12, à partir de cartouches dans des appareils de mélange automatique opérés par machine.

15. Utilisation d'un matériau de prise d'empreinte dentaire en élastomère à deux composants, indéformable et stable au stockage selon l'une des revendications 1 à 12, au cours d'un malaxage manuel.

16. Utilisation d'un matériau de prise d'empreinte dentaire en élastomère à deux composants, indéformable et stable au stockage selon l'une des revendications 1 à 12 dans la prise d'empreinte de correction ou dans la prise d'empreinte à double mélange.

17. Kit dentaire, comportant soit
• un matériau de prise d'empreinte dentaire en élastomère à deux composants, indéformable et stable au stockage, selon l'une des revendications 1 à 12,
• une ou plusieurs canules de mélange,
• facultativement des cuillères de prise d'empreinte ou facultativement un matériau destiné à fabriquer une cuillère de prise d'empreinte individuelle,
• facultativement un matériau de prise d'empreinte de correction faiblement, moyennement ou hautement visqueux,
ou
• un matériau de prise d'empreinte dentaire en élastomère à deux composants, indéformable et stable au stockage selon l'une des revendications 1 à 12,
• une ou plusieurs cuillères de dosage,
• facultativement des cuillères de prise d'empreinte ou facultativement un matériau destiné à fabriquer une cuillère de prise d'empreinte individuelle,
• facultativement un matériau de prise d'empreinte de correction faiblement, moyennement ou hautement visqueux,
• facultativement un bloc de mélange.

18. Procédé pour la fabrication d'une empreinte dentaire, comportant les étapes suivantes :
• mise à disposition d'un matériau de prise d'empreinte dentaire en élastomère à deux composants, indéformable et stable au stockage selon l'une des revendications 1 à 12,
• mise à disposition d'un moule approprié,
• remplissage du moule avec le matériau de prise d'empreinte dentaire en élastomère à deux composants, indéformable et stable au stockage,
• positionnement du moule rempli dans la bouche du patient,
• durcissement du matériau de prise d'empreinte dentaire en élastomère à deux composants, indéformable et stable au stockage,
• retrait de l'empreinte obtenue hors de la bouche du patient,
• le cas échéant, découpage, rognage ou autre traitement de l'empreinte obtenue.
